# EUROPEAN PATENT SPECIFICATION

(11) **EP 3 233 915 B1**
(45) Date of publication and mention of the grant of the patent: **28.08.2024**
(21) Application number: 15813341.3
(22) Date of filing: 15.12.2015
(51) Int. Cl.: C07K 16/24, A61P 29/00, A61P 35/00

(54) **ANTIBODIES FOR IL-17C**
ANTIKÖRPER FÜR IL-17C
ANTICORPS POUR IL-17C

(30) Priority: 15.12.2014 EP 14197883
(43) Date of publication of application: 25.10.2017
(73) Proprietor: MorphoSys AG, 82152 Planegg (DE)
(72) Inventor: KLATTIG, Jürgen, 82152 Martinsried / Planegg (DE); VANDEGHINSTE, Nick Ernest René, B-2800 Mechelen (BE); GARCIA, Teresa, F-93230 Romainville (FR)
(74) Representative: Spiller, Stephan
(86) International application number: PCT/EP2015/079856
(87) International publication number: WO 2016/096896

(56) References cited:
- WO-A1-2007/047738
- WO-A1-2013/057241
- WO-A2-99/60127
- XINYANG SONG ET AL: "Alterations in the Microbiota Drive Interleukin-17C Production from Intestinal Epithelial Cells to Promote Tumorigenesis", IMMUNITY, vol. 40, no. 1, 1 January 2014 (2014-01-01), pages 140 - 152, XP055190777, ISSN: 1074-7613, DOI: 10.1016/j.immuni.2013.11.018
- RAJITA PAPPU ET AL: "The IL-17 Family Cytokines in Immunity and Disease", JOURNAL OF CLINICAL IMMUNOLOGY, KLUWER ACADEMIC PUBLISHERS-PLENUM PUBLISHERS, NE, vol. 30, no. 2, 23 February 2010 (2010-02-23), pages 185 - 195, XP019791216, ISSN: 1573-2592
- LI H ET AL: "Cloning and characterization of the iL-17B and iL-17C, two members of the iL-17 cytokine family", PROCEEDINGS OF THE NATIONAL ACADEMY OF SCIENCES, NATIONAL ACADEMY OF SCIENCES, US, vol. 97, no. 2, 18 January 2000 (2000-01-18), pages 773 - 778, XP002139729, ISSN: 0027-8424, DOI: 10.1073/PNAS.97.2.773
- VLADIMIR RAMIREZ-CARROZZI ET AL: "IL-17C regulates the innate immune function of epithelial cells in an autocrine manner", NATURE IMMUNOLOGY, NATURE PUBLISHING GROUP, GB, vol. 12, no. 12, 1 December 2011 (2011-12-01), pages 1159 - 1166, XP002666790, ISSN: 1529-2908, [retrieved on 20111012], DOI: 10.1038/NI.2156

## Description

### Field of the invention

The present application generally relates to antibodies or antibody fragments which interact with IL-17C. In particular, it relates to antibodies or antibody fragments that form a complex consisting of said antibody or antibody fragment and one IL-17C homodimer.

### Background

IL-17C is a secreted homodimer of the IL17 protein family. *In vitro* it has been shown that IL-17C stimulates the release of TNF-α and IL-1β from the monocytic cell line THP-1 (Li etal. (2000) Proc. Natl. Acad. Sci. U. S. A. 97, 773-8). IL-17C can induce the mRNA expression of inflammatory cytokines such as IL-1β, IL-6 and IL-23 in peritoneal exudates cells (PECS) and the 3T3 cell line (Yamaguchi et al. (2007) J. Immunol 179, 7128-36).

As a functional receptor specific for IL-17C, IL-17RE was identified and *IL-17C*^{*-*/*-*} mice were found to be resistant to experimental autoimmune encephalomyelitis (EAE), indicating the role of IL-17C as a proinflammtory cytokine (Chang et al. (2011) Immunity 35, 611-621). IL-17C activates downstream signaling through a receptor complex of IL-17RE and IL-17RA for the induction of genes encoding antibacterial molecules to combat host mucosal immunity mediated by intestinal pathogens (Song et al. (2011) Nature Immunology 12, 12). IL-17C and its receptor were further described to be prominently expressed on mucosal and epithelial cells and therefore provide a mechanism by which the epithelium participates in host defense (Ramirez-Carrozzi et al. (2011) Nature Immunology 12, 12).

WO 1999/060127 is the first disclosure describing the cloning of IL-17C (PRO1122). So far IL-17C was postulated to be involved in the progression of several inflammatory disorders but only recently in WO 2013/057241 it was experimentally evaluated that inhibition of IL-17C is a promising approach to treat inflammatory disorders. However, respective antibodies used in WO 2013/057241 were surrogates, specific for mouse IL-17C, and were shown not to be reactive to human IL-17C at all. In addition, in recent disclosures relevance of I L-17C in the progression of specific tumours and cancerous tissues was shown (Xinyang Song (2014) Immunity 40, 140-152).

Meanwhile several antibodies such as polyclonal sera or monoclonal antibodies derived from immunized mice are available as research tool-antibodies, including e.g. MAB23061 and MAB2306 from R&D Systems, the anti-IL-17C antibodies available from Abnova (Walnut, CA, USA; Catalog #H00027189-B01P, #H00027189-D01 and #H00027189-D01P) or the anti-IL-17C antibodies available from antibodies-online GmbH (Aachen, Germany; Catalog #ABIN525892, #ABIN327411, #ABIN525893, #ABIN221340, #ABIN221341, #ABIN221342 and #ABIN525891).

However, such antibodies are either polyclonal sera or specifically bind to mouse IL-17C such as the antibodies used in WO 2013/057241 and are not applicable for human therapy.

Accordingly, a need exists to study and identify methods and compositions that alter IL-17C mediated signalling in human and ameliorating IL-17C related diseases or disorders.

### Summary of the invention

The applicant for the first time discloses antibodies or antibody fragments that bind to human IL-17C. More specifically the disclosed antibodies or antibody fragments bivalently bind to an IL-17C homodimer. Even more specifically the antibodies or antibody fragments bivalently bind to an IL-17C homodimer and form a complex consisting of said antibody or antibody fragment and one IL-17C homodimer. In one embodiment of the present disclosure, the antibody or fragment thereof is an IL-17C antagonist. In another embodiment of the present disclosure, the antibody or antibody fragments block the binding of IL-17C to IL17RE.

The disclosed antibodies or antibody fragments are capable to interact with one IL-17C homodimer by simultaneous (bivalent) binding of both antigen-binding sites from the VH/VL pairs. Therefore such antibodies bind to specific epitopes of IL-17C that are accessible twice on homodimeric IL-17C and can be bivalently bound by the antibodies disclosed herein. The strong avidity effect as a result of the bivalent binding leads to the formation of very stable complexes between one IL-17C homodimer and such antibodies or antibody fragments. In addition, the disclosed antibodies or antibody fragments inhibit interaction of IL-17C with its receptor and by the bivalent binding the disclosed antibodies or antibody fragments concurrently block both receptor interaction regions of the IL-17C homodimer accompanied with an outstanding efficient abrogation of IL-17C-mediated signaling. Consequently an antibody as disclosed herein is outstanding in its antagonistic activity based on its surprising binding properties.

Thus, the disclosed antibodies or antibody fragments are therefore special in terms of effectiveness and provide well suited and promising compounds for clinical development.

Provided herein is an antibody or antibody fragment specific for IL-17C wherein said antibody or antibody fragment bivalently binds to an IL-17C homodimer and forms a complex consisting of said antibody or antibody fragment and one IL-17C homodimer, and wherein the antibody or antibody fragment binds to the region PVLRPEEVL (SEQ ID NO.: 27) on IL-17C.

In one aspect said antibody or antibody fragment binds to human IL-17C.

In another aspect said antibody or antibody fragment blocks the binding of IL-17C to IL17RE.

In one embodiment of the present disclosure, the antibody or fragment thereof is a monoclonal antibody or antibody fragment. In one embodiment of the present disclosure, the antibody or fragment thereof is of the IgG isotype. In one embodiment the antibody fragment is a bivalent antibody fragment.

In another embodiment said antibody or antibody fragment is an isolated antibody or antibody fragment. In another embodiment said antibody or antibody fragment is a recombinant antibody or antibody fragment

In another aspect the disclosure pertains to the medical use of the disclosed antibodies. In another aspect the disclosure pertains to the use of the disclosed antibodies for the treatment of inflammatory disorder or cancer in humans.

In another embodiment the antibody or antibody fragment is a human, humanized or chimeric antibody or antibody fragment. In another embodiment said antibody or antibody fragment comprises a human heavy chain constant region and a human light chain constant region. In another embodiment said antibody or antibody fragment is a recombinant antibody or antibody fragment. In another embodiment said recombinant antibody or antibody fragment is a recombinant human antibody or antibody fragment.

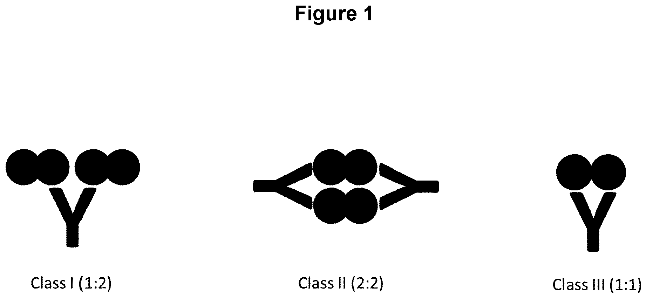

In one embodiment, the antibodies or antibody fragments specific for IL-17C form a complex with an IL-17C homodimer wherein said complex has a molecular weight lower than 200kDa. In one embodiment, said antibodies or antibody fragments form a complex with an IL-17C homodimer, wherein at least 50% of said formed complexes have a molecular weight lower than 200kDa. In further embodiments, said antibodies or antibody fragments form a complex with an IL-17C homodimer, wherein at least 60%, at least 70%, at least 80%, at least 90% of said formed complexes have a molecular weight lower than 200kDa. In another embodiment said complex is the class III complex as described herein in Example 2, section 7 (Figure 1). In another embodiment said complex is formed in solution and consists of an antibody and one IL-17C homodimer.

In another embodiment, the complex formed by an antibody specific for IL-17C and one IL-17C homodimer is assessed by size exclusion chromatography.

In one embodiment the present disclosure relates to the use of antibodies or antibody fragments that bivalently bind to an IL-17C homodimer for the treatment of a disorder or condition associated with the undesired presence of IL-17C.

In another embodiment the present disclosure relates to a nucleic acid composition comprising a nucleic acid sequence or a plurality of nucleic acid sequences encoding the antibody or antibody fragment as disclosed herein.

In another embodiment the present disclosure relates to vector composition comprising a vector or a plurality of vectors comprising the nucleic acid sequence or plurality of nucleic acid sequences encoding the antibody or antibody fragment as disclosed herein.
In another embodiment the present disclosure relates to a cell comprising said vector composition.

In one embodiment the present disclosure relates to a pharmaceutical composition comprising said antibody or antibody and a pharmaceutically acceptable carrier or excipient.

In another embodiment the present disclosure relates to the use of said pharmaceutical composition for the treatment of a disorder or condition associated with the undesired presence of IL-17C.

There is utility in the claimed antibodies or antibody fragments. Furthermore, there is utility in the claimed method to identify such antibodies or fragments.

Utilization of the claimed antibodies or antibody fragments is to alter the biological activity of human IL-17C. In particular the claimed antibodies or antibody fragments are intended for therapeutic use, such as the treatment of inflammatory disorders like e.g. rheumatoid arthritis, psoriasis, pulmonary inflammation and/or COPD.

### Figure legends

**Figure 1****:** Potential binding modes of IL-17C antibodies are illustrated. Depending on the binding mode of the antibody mainly one out of three complexes is formed, wherein either two homodimers are associated with one antibody (class I), two homodimers are associated with two antibodies (class II) or one homodimer is associated with one antibody (class III). The formed complexes differ in their molecular weight wherein Class I complexes have a molecular weight of -270 kDa, Class II complexes have a molecular weight of -380 kDa and Class III complexes have a molecular weight of ~190 kDa.
**Figure 2****:** Size exclusion chromatography of antibody mab_1 analysing complex formation of mab_1 with human IL-17C and cynomolgus IL-17C. Mab_1 can be clearly identified to form a class III complex with one homodimer of human IL-17C and cynomolgus IL-17C, respectively.
**Figure 3****:** Size exclusion chromatography of antibody mab_8 analysing complex formation of mab_8 with human IL-17C. Complex formation with one IL-17C homodimer can be observed.
**Figure 4** **and** **Figure 5****:** Both figures exemplify 5 additional antibodies sharing the same binding mode in size exclusion chromatography as identified for mab_1 and mab_8.

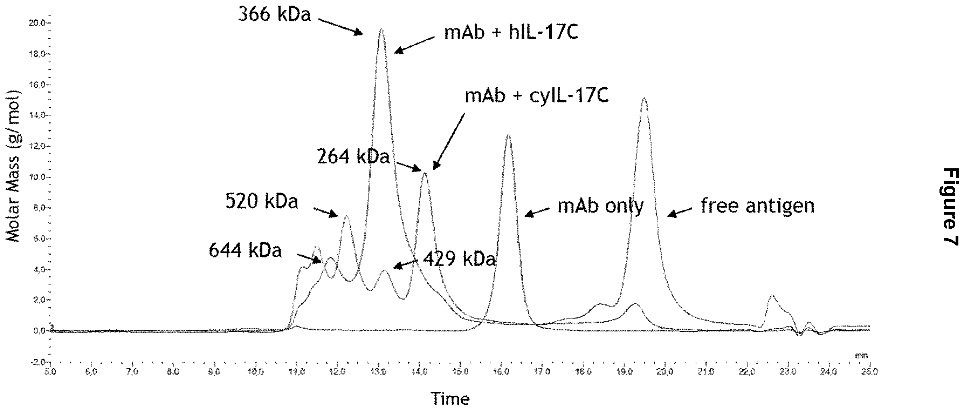

**Figure 6** **and** **Figure 7****:** exemplify two out of many antibodies, which do not share the binding mode identified for mab_1 and mab_8. The antibody in Figure 6 mainly forms class II complexes (~365kDa) with human IL-17C and mainly class I complexes (~253kDa) with cynomolgus IL-17C. Also the antibody in Figure 7 mainly forms class II complexes (~366kDa) with human IL-17C and mainly class I complexes (~264kDa) with cynomolgus IL-17C.
**Figure 8A** **and** **8B****:** show the coverage maps of the HDX MS epitope mapping of the antibodies mab_1 **(****Figure 8A****)** and mab_8 **(****Figure 8B****).** The region PVLRPEEVL (SEQ ID No.: 27, aa 89-97 of SEQ ID No.: 1) was identified as the main epitope on human IL-17C for both antibodies.

### Detailed description of the invention

The disclosure pertains to a number of antibodies or antibody fragments that recognize at least a specific region of IL-17C. In one aspect, the antibodies or antibody fragments of the present disclosure bind to IL-17C and form a complex with an IL-17C homodimer. Preferably, said antibodies or antibody fragments bivalently bind to an IL-17C homodimer and form a complex consisting of said antibody or antibody fragment and one IL-17C homodimer. Preferably, said antibodies or antibody fragments bind to human IL-17C.

### Definitions:

The term "IL-17C" refers to a protein known as interleukin 17C.

Human IL-17C has the amino acid sequence of (UniProt Q9P0M4):

MTLLPGLLFLTWLHTCLAHHDPSLRGHPHSHGTPHCYSAEELPLGQAPPHLLA
RGAKWGQALPVALVSSLEAASHRGRHERPSATTQCPVLRPEEVLEADTHQR
SISPWRYRVDTDEDRYPQKLAFAECLCRGCIDARTGRETAALNSVRLLQSLLV
LRRRPCSRDGSGLPTPGAFAFHTEFIHVPVGCTCVLPRSV (SEQ ID No.: 1)

PVLRPEEVL (SEQ ID No.: 27) corresponds to amino acids aa 89-97 of SEQ ID No.: 1.
VLRPEEVL (SEQ ID No.: 28) corresponds to amino acids aa 90-97 of SEQ ID No.: 1.

Mouse IL-17C has the amino acid sequence of (UniProt Q8K4C5):

MSLLLLGWLPTGMTHQDPPSWGKPRSHRTLRCYSAEELSHGQAPPHLLTRS
ARWEQALPVALVASLEATGHRRQHEGPLAGTQCPVLRPEEVLEADTHERSIS

PWRYRIDTDENRYPQKLAVAECLCRGCINAKTGRETAALNSVQLLQSLLVLRR
QPCSRDGTADPTPGSFAFHTEFIRVPVGCTCVLPRSTQ (SEQ ID No.: 2)

Cynomolgus IL-17C has the amino acid sequence of (XP_005592825.1):

MTLLPGLLFLTWLHACLAHQDPFLRGHPHTHGTPRCYSAEELPLGQAPPHLLA
RGAKWGQALPVALVSSLEAAGHRRRHDRPSAATQCPVLRPEEVLEADTHQR
SISPWRYRVDTDEDRYPQKLAFAECLCRGCIDPRTGRETAALNSVRLLQSLLV
LRRRPCSRDGSGLPTPGAFAFHTEFIRVPVGCTCVLPRSV (SEQ ID No.: 3)

The term "homodimer" refers to two identical molecules linked together, such as for example by disulfide linkages or non-covalent interactions.

The term "IL17RA" refers to a protein known as interleukin 17 receptor A. Human IL17RA has the amino acid sequence of (UniProt Q96F46):

MGAARSPPSAVPGPLLGLLLLLLGVLAPGGASLRLLDHRALVCSQPGLNCTVKNSTC
LDDSWIHPRNLTPSSPKDLQIQLHFAHTQQGDLFPVAHIEWTLQTDASILYLEGAELS
VLQLNTNERLCVRFEFLSKLRHHHRRWRFTFSHFVVDPDQEYEVTVHHLPKPIPDG
DPNHQSKNFLVPDCEHARMKVTTPCMSSGSLWDPNITVETLEAHQLRVSFTLWNES
THYQILLTSFPHMENHSCFEHMHHIPAPRPEEFHQRSNVTLTLRNLKGCCRHQVQIQ
PFFSSCLNDCLRHSATVSCPEMPDTPEPIPDYMPLWVYWFITGISILLVGSVILLIVCM
TWRLAGPGSEKYSDDTKYTDGLPAADLIPPPLKPRKVWIIYSADHPLYVDVVLKFAQ
FLLTACGTEVALDLLEEQAISEAGVMTWVGRQKQEMVESNSKIIVLCSRGTRAKWQ
ALLGRGAPVRLRCDHGKPVGDLFTAAMNMILPDFKRPACFGTYVVCYFSEVSCDGD
VPDLFGAAPRYPLMDRFEEVYFRIQDLEMFQPGRMHRVGELSGDNYLRSPGGRQL
RAALDRFRDWQVRCPDWFECENLYSADDQDAPSLDEEVFEEPLLPPGTGIVKRAPL
VREPGSQACLAIDPLVGEEGGAAVAKLEPHLQPRGQPAPQPLHTLVLAAEEGALVA
AVEPGPLADGAAVRLALAGEGEACPLLGSPGAGRNSVLFLPVDPEDSPLGSSTPMA
SPDLLPEDVREHLEGLMLSLFEQSLSCQAQGGCSRPAMVLTDPHTPYEEEQRQSV
QSDQGYISRSSPQPPEGLTEMEEEEEEEQDPGKPALPLSPEDLESLRSLQRQLLFR
QLQKNSGWDTMGSESEGPSA (SEQ ID No.: 4)

The term "IL17RE" refers to a protein known as interleukin 17 receptor E. Human IL17RE has the amino acid sequence of (UniProt Q8NFR9):

MGSSRLAALLLPLLLIVIDLSDSAGIGFRHLPHWNTRCPLASHTDDSFTGSSAYIPCRT
WWALFSTKPWCVRVWHCSRCLCQHLLSGGSGLQRGLFHLLVQKSKKSSTFKFYRR
HKMPAPAQRKLLPRRHLSEKSHHISIPSPDISHKGLRSKRTQPSDPETWESLPRLDS
QRHGGPEFSFDLLPEARAIRVTISSGPEVSVRLCHQWALECEELSSPYDVQKIVSGG
HTVELPYEFLLPCLCIEASYLQEDTVRRKKCPFQSWPEAYGSDFWKSVHFTDYSQH
TQMVMALTLRCPLKLEAALCQRHDWHTLCKDLPNATARESDGWYVLEKVDLHPQL
CFKFSFGNSSHVECPHQTGSLTSWNVSMDTQAQQLILHFSSRMHATFSAAWSLPG
LGQDTLVPPVYTVSQARGSSPVSLDLIIPFLRPGCCVLVWRSDVQFAWKHLLCPDVS
YRHLGLLILALLALLTLLGVVLALTCRRPQSGPGPARPVLLLHAADSEAQRRLVGALA
ELLRAALGGGRDVIVDLWEGRHVARVGPLPWLWAARTRVAREQGTVLLLWSGADL
RPVSGPDPRAAPLLALLHAAPRPLLLLAYFSRLCAKGDIPPPLRALPRYRLLRDLPRL
LRALDARPFAEATSWGRLGARQRRQSRLELCSRLEREAARLADLG (SEQ ID No.: 5)

Murine IL17RE has the amino acid sequence of (UniProt Q8BH06):

MGSPRLAALLLSLPLLLIGLAVSARVACPCLRSWTSHCLLAYRVDKRFAGLQWGWF
PLLVRKSKSPPKFEDYWRHRTPASFQRKLLGSPSLSEESHRISIPSSAISHRGQRTK
RAQPSAAEGREHLPEAGSQKCGGPEFSFDLLPEVQAVRVTIPAGPKASVRLCYQW
ALECEDLSSPFDTQKIVSGGHTVDLPYEFLLPCMCIEASYLQEDTVRRKKCPFQSWP
EAYGSDFWQSIRFTDYSQHNQMVMALTLRCPLKLEASLCWRQDPLTPCETLPNATA
QESEGWYILENVDLHPQLCFKFSFENSSHVECPHQSGSLPSWTVSMDTQAQQLTL
HFSSRTYATFSAAWSDPGLGPDTPMPPVYSISQTQGSVPVTLDLIIPFLRQENCILVW
RSDVHFAWKHVLCPDVSHRHLGLLILALLALTALVGVVLVLLGRRLLPGSGRTRPVLL
LHAADSEAQRRLVGALAELLRTALGGGRDVIVDLWEGTHVARIGPLPWLWAARERV
AREQGTVLLLWNCAGPSTACSGDPQAASLRTLLCAAPRPLLLAYFSRLCAKGDIPRP
LRALPRYRLLRDLPRLLRALDAQPATLASSWSHLGAKRCLKNRLEQCHLLELEAAKD
DYQGSTNSPCGFSCL (SEQ ID No.: 6)

The term "complex"' means the entity created when two or more compounds bind to, contact, or associate with each other. Herein a complex is formed between an antibody or an antibody fragment and its antigen.

An "antagonist of IL-17C" and an "IL-17C antagonist", as used herein, refers to any molecule which inhibits the activity or function of IL-17C. The term IL-17C antagonist includes, but is not limited to, antibodies or antibody fragments specifically binding to IL-17C. Preferably, an IL-17C antagonist in the present disclosure is an antibody specific for human IL-17C. Such an antibody may be of any type, such as a murine, a rat, a chimeric, a humanized or a human antibody.

The term "antibody" as used herein refers to a protein comprising at least two heavy (H) chains and two light (L) chains inter-connected by disulfide bonds which interacts (e.g., by binding, steric hindrance, stabilizing spatial distribution) with an antigen. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FR's arranged from amino-terminus to carboxy-terminus in the following order: FR1, CDR1, FR2, CDR2, FR3, CDR3, and FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (Clq) of the classical complement system. The term "antibody" includes for example, monoclonal antibodies, human antibodies, humanized antibodies, camelised antibodies and chimeric antibodies. The antibodies can be of any isotype (e.g., IgG, IgE, IgM, IgD, IgA and IgY), class (e.g., IgG1, IgG2, IgG3, IgG4, lgA1 and IgA2) or subclass. Both the light and heavy chains are divided into regions of structural and functional homology.

The phrase "antibody fragment", as used herein, refers to one or more portions of an antibody that retain the ability to specifically interact with (e.g., by binding, steric hindrance, stabilizing spatial distribution) an antigen. Examples of binding fragments include, but are not limited to, a Fab fragment, a monovalent fragment consisting of the VL, VH, CL and CH1 domains; a F(ab)2 fragment, a bivalent fragment comprising two Fab fragments linked by a disulfide bridge at the hinge region; a Fd fragment consisting of the VH and CH1 domains; a Fv fragment consisting of the VL and VH domains of a single arm of an antibody; a dAb fragment (Ward et al., (1989) Nature 341:544-546), which consists of a VH domain; and an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see e.g., Bird et al., (1988) Science 242:423-426; and Huston et al., (1988) Proc. Natl. Acad. Sci. 85:5879-5883). Such single chain antibodies are also intended to be encompassed within the term "antibody fragment". These antibody fragments are obtained using conventional techniques known to those of skill in the art, and the fragments are screened for utility in the same manner as are intact antibodies. Antibody fragments can also be incorporated into single domain antibodies, maxibodies, minibodies, intrabodies, diabodies, triabodies, tetrabodies, v-NAR and bis-scFv (see, e.g., Hollinger and Hudson, (2005) Nature Biotechnology 23:1126-1136). Antibody fragments can be grafted into scaffolds based on polypeptides such as Fibronectin type III (Fn3) (see U.S. Pat. No. 6,703,199, which describes fibronectin polypeptide monobodies). Antibody fragments can be incorporated into single chain molecules comprising a pair of tandem Fv segments (VH-CH1-VH-CH1) which, together with complementary light chain polypeptides, form a pair of antigen-binding sites (Zapata et al., (1995) Protein Eng. 8:1057-1062; and U.S. Pat. No. 5,641,870).

The term "antigen binding site" refers to the part of the antibody or antibody fragment that comprises the area that specifically binds to an antigen. An antigen binding site may be provided by one or more antibody variable domains. Preferably, an antigen binding site is comprised within the associated VH and VL of an antibody or antibody fragment.

A "human antibody" or "human antibody fragment", as used herein, includes antibodies and antibody fragments having variable regions in which both the framework and CDR regions are derived from sequences of human origin. Furthermore, if the antibody contains a constant region, the constant region also is derived from such human sequences, e.g., human germline sequences, or mutated versions of human germline sequences or antibody containing consensus framework sequences derived from human framework sequences analysis, for example, as described in Knappik et al., (2000) J Mol Biol 296:57-86). The structures and locations of immunoglobulin variable domains, e.g., CDRs, may be defined using well known numbering schemes, e.g., the Kabat numbering scheme, the Chothia numbering scheme, or a combination of Kabat and Chothia (see, e.g., Sequences of Proteins of Immunological Interest, U.S. Department of Health and Human Services (1991), eds. Kabat et al.; Lazikani et al., (1997) J. Mol. Bio. 273:927-948); Kabat et al., (1991) Sequences of Proteins of Immunological Interest, 5th edit., NIH Publication no. 91-3242 U.S. Department of Health and Human Services; Chothia et al., (1987) J. Mol. Biol. 196:901-917; Chothia et al., (1989) Nature 342:877-883; and Al-Lazikani et al., (1997) J. Mol. Biol. 273:927-948.

A "humanized antibody" or functional humanized antibody fragment is defined herein as one that is (i) derived from a non-human source (e.g., a transgenic mouse which bears a heterologous immune system), which antibody is based on a human germline sequence or (ii) CDR-grafted, wherein the CDRs of the variable domain are from a non-human origin, while one or more frameworks of the variable domain are of human origin and the constant domain (if any) is of human origin.

The term "chimeric antibody" or functional chimeric antibody fragment is defined herein as an antibody molecule which has constant antibody regions derived from, or corresponding to, sequences found in one species and variable antibody regions derived from another species. Preferably, the constant antibody regions are derived from, or corresponding to, sequences found in humans, e.g. in the human germ line or somatic cells, and the variable antibody regions (e.g. VH , VL , CDR or FR regions) are derived from sequences found in a non-human animal, e.g. a mouse, rat, rabbit or hamster.

The term "isolated" refers to a compound which can be e.g. an antibody or antibody fragment that is substantially free of other antibodies or antibody fragments having different antigenic specificities. Moreover, an isolated antibody or antibody fragment may be substantially free of other cellular material and/or chemicals. Thus, in some aspects, antibodies provided are isolated antibodies which have been separated from antibodies with a different specificity. An isolated antibody may be a monoclonal antibody. An isolated antibody may be a recombinant monoclonal antibody. An isolated antibody that specifically binds to an epitope, isoform or variant of a target may, however, have cross-reactivity to other related antigens, e.g., from other species (e.g., species homologs).

The term "recombinant antibody", as used herein, includes all antibodies that are prepared, expressed, created or segregated by recombinant means, such as antibodies isolated from an animal (e.g., a mouse) that is transgenic or transchromosomal for human immunoglobulin genes or a hybridoma prepared therefrom, antibodies isolated from a host cell transformed to express the antibody, antibodies selected and isolated from a recombinant, combinatorial human antibody library, and antibodies prepared, expressed, created or isolated by any other means that involve splicing of all or a portion of a human immunoglobulin gene, sequences to other DNA sequences. Preferably, such recombinant antibodies have variable regions in which the framework and CDR regions are derived from human germline immunoglobulin sequences. In certain embodiments, however, such recombinant human antibodies can be subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo. A recombinant antibody may be a monoclonal antibody. In an embodiment, the antibodies and antibody fragment disclosed herein are isolated from the Ylanthia^{®} antibody library as disclosed in US 13/321,564 or US 13/299,367, which both herein are incorporated by reference.

The term "monoclonal antibody" as used herein refers to a preparation of antibody molecules of single molecular composition. A monoclonal antibody composition displays a unique binding site having a unique binding specificity and affinity for particular epitopes.

As used herein, an antibody "binds specifically to", "specifically binds to", is "specific to/for" or "specifically recognizes" an antigen if such antibody is able to discriminate between such antigen and one or more reference antigen(s), since binding specificity is not an absolute, but a relative property. The reference antigen(s) may be one or more closely related antigen(s), which are used as reference points, e.g. IL17A or IL17B. In its most general form (and when no defined reference is mentioned), "specific binding" is referring to the ability of the antibody to discriminate between the antigen of interest and an unrelated antigen, as determined, for example, in accordance with one of the following methods. Such methods comprise, but are not limited to Western blots, ELISA-, RIA-, ECL-, IRMA-tests and peptide scans. For example, a standard ELISA assay can be carried out. The scoring may be carried out by standard color development (e.g. secondary antibody with horseradish peroxide and tetramethyl benzidine with hydrogen peroxide). The reaction in certain wells is scored by the optical density, for example, at 450 nm. Typical background (=negative reaction) may be 0.1 OD; typical positive reaction may be 1 OD. This means the difference positive/negative can be more than 10-fold. Typically, determination of binding specificity is performed by using not a single reference antigen, but a set of about three to five unrelated antigens, such as milk powder, BSA, transferrin or the like. Additionally, "specific binding" may relate to the ability of an antibody to discriminate between different parts of its target antigen, e.g. different domains or regions of IL-17C or the receptor of IL-17C, or between one or more key amino acid residues or stretches of amino acid residues of IL-17C or the receptor of IL-17C.

The term "avidity" is used to describe the combined strength of multiple bond interactions between proteins. Avidity is distinct from affinity which describes the strength of a single bond. As such, avidity is the combined synergistic strength of bond affinities (functional affinity) rather than the sum of bonds. With the antibodies of the present disclosure, both antigen-binding sites from the VH/VL pairs simultaneously interact with one IL-17C homodimer. Whilst each single binding interaction may be readily broken (depending on the relative affinity), because many binding interactions are present at the same time, transient unbinding of a single site does not allow the molecule to diffuse away, and binding of that site is likely to be reinstated. The overall effect is synergistic, strong binding of antigen to antibody.

As used herein, the term "affinity" refers to the strength of interaction between the polypeptide and its target at a single site. Within each site, the binding region of the polypeptide interacts through weak non-covalent forces with its target at numerous sites; the more interactions, the stronger the affinity.

The term "K_{D}", as used herein, refers to the dissociation constant, which is obtained from the ratio of K_{d} to Kₐ (i.e. K_{d}/Kₐ) and is expressed as a molar concentration (M). K_{D} values for antigen binding moieties like e.g. monoclonal antibodies can be determined using methods well established in the art. Methods for determining the K_{D} of an antigen binding moiety like e.g. a monoclonal antibody are SET (soluble equilibrium titration) or surface plasmon resonance using a biosensor system such as a Biacore^{®} system. In the present disclosure an antibody specific to IL-17C typically has a dissociation rate constant (K_{D}) (k_{off}/kₒₙ) of less than 5×10⁻²M, less than 10⁻²M, less than 5×10⁻³M, less than 10⁻³M, less than 5×10⁻⁴M, less than 10⁻⁴M, less than 5×10⁻⁵M, less than 10⁻⁵M, less than 5×10⁻⁶M, less than 10⁻⁶M, less than 5x10-'M, less than 10-'M, less than 5×10⁻⁸M, less than 10⁻⁸M, less than 5×10⁻⁹M, less than 10⁻⁹M, less than 5x10-10M, less than 10⁻¹⁰M, less than 5×10⁻¹¹M, less than 10⁻¹¹M, less than 5×10⁻¹²M, less than 10⁻¹²M, less than 5×10⁻¹³M, less than 10⁻¹³M, less than 5×10⁻¹⁴M, less than 10⁻¹⁴M, less than 5×10⁻¹⁵M, or less than 10⁻¹⁵M or lower.

The term "bivalent molecule" as used herein refers to a molecule that has two antigen-binding sites. In some embodiments, a bivalent molecule of the present invention is a bivalent antibody or a bivalent fragment thereof. In some embodiments, a bivalent molecule of the present invention is a bivalent antibody. In some embodiments, a bivalent molecule of the present invention is an IgG. In general monoclonal antibodies have a bivalent basic structure. IgG and IgE have only one bivalent unit, while IgA and IgM consist of multiple bivalent units (4 and 10, respectively) and thus have higher valencies. This bivalency increases the avidity of antibodies for antigens.

The terms "bivalent binding" or "bivalently binds to" as used herein refer to the binding of both antigen-binding sites of a bivalent molecule to its antigen. Preferably both antigen-binding sites of a bivalent molecule share the same antigen specificity.

"Cross competes" means the ability of an antibody, antibody fragment or other antigen-binding moieties to interfere with the binding of other antibodies, antibody fragments or antigen-binding moieties to a specific antigen in a standard competitive binding assay. The ability or extent to which an antibody, antibody fragment or other antigen-binding moieties is able to interfere with the binding of another antibody, antibody fragment or antigen-binding moieties to a specific antigen, and, therefore whether it can be said to cross-compete according to the invention, can be determined using standard competition binding assays. One suitable assay involves the use of the Biacore technology (e.g. by using the BIAcore 3000 instrument (Biacore, Uppsala, Sweden)), which can measure the extent of interactions using surface plasmon resonance technology. Another assay for measuring cross-competing uses an ELISA-based approach. A high throughput process for "epitope binning" antibodies based upon their cross-competition is described in International Patent Application No. WO 2003/48731. Cross-competition is present if the antibody or antibody fragment under investigation reduces the binding of one of the antibodies described in Table 1 to IL-17C by 60% or more, specifically by 70% or more and more specifically by 80% or more and if one of the antibodies described in Table 1 reduces the binding of said antibody or antibody fragment to IL-17C by 60% or more, specifically by 70% or more and more specifically by 80% or more.

The term "epitope" includes any proteinacious region which is specifically recognized by an antibody or fragment thereof or a T-cell receptor or otherwise interacts with a molecule. Generally epitopes are of chemically active surface groupings of molecules such as amino acids or carbohydrate or sugar side chains and generally may have specific three-dimensional structural characteristics, as well as specific charge characteristics. As will be appreciated by one of skill in the art, practically anything to which an antibody can specifically bind could be an epitope. An epitope can comprise those residues to which the antibody binds and may be "linear" or "conformational." The term "linear epitope" refers to an epitope wherein all of the points of interaction between the protein and the interacting molecule (such as an antibody) occur linearly along the primary amino acid sequence of the protein (continuous). The term "conformational epitope" refers to an epitope in which discontinuous amino acids that come together in three dimensional conformations. In a conformational epitope, the points of interaction occur across amino acid residues on the protein that are separated from one another. For example, an epitope can be one or more amino acids within a stretch of amino acids as shown by peptide mapping or HDX, or one or more individual amino acids as shown by X-ray crystallography.

"Binds the same epitope as" means the ability of an antibody, antibody fragment or other antigen-binding moiety to bind to a specific antigen and having the same epitope as the exemplified antibody. The epitopes of the exemplified antibody and other antibodies can be determined using epitope mapping techniques. Epitope mapping techniques are well known in the art. For example, conformational epitopes are readily identified by determining spatial conformation of amino acids such as by, e.g., hydrogen/deuterium exchange, x-ray crystallography and two-dimensional nuclear magnetic resonance.

Compositions of the invention may be used for therapeutic or prophylactic applications. The invention, therefore, includes a pharmaceutical composition containing an inventive antibody (or functional antibody fragment) and a pharmaceutically acceptable carrier or excipient therefor. In a related aspect, the invention provides a method for treating an inflammatory disorder. Such method contains the steps of administering to a subject in need thereof an effective amount of the pharmaceutical composition that contains an inventive antibody as described or contemplated herein.

The present disclosure provides therapeutic methods comprising the administration of a therapeutically effective amount of an IL-17C antibody as disclosed to a subject in need of such treatment. A "therapeutically effective amount" or "effective amount", as used herein, refers to the amount of an IL-17C antibody necessary to elicit the desired biological response. In accordance with the subject invention, the therapeutic effective amount is the amount of an IL-17C antibody necessary to treat and/or prevent a disease.

The terms "inflammatory disorder" or "inflammatory disease" are used interchangeably and as used herein refer to any abnormality associated with inflammation. Inflammatory disorders may be chronic or acute and include autoimmune diseases.

"Subject", as used in this context refers to any mammal, including rodents, such as mouse or rat, and primates, such as cynomolgus monkey (*Macaca fascicularis*), rhesus monkey (*Macaca mulatta*) or humans (*Homo sapiens*). Preferably the subject is a primate, most preferably a human.

### Embodiments:

In one embodiment the present disclosure refers to an antibody or antibody fragment specific for IL-17C wherein said antibody or antibody fragment bivalently binds to an IL-17C homodimer and forms a complex consisting of said antibody or antibody fragment and one IL-17C homodimer.

In one embodiment the present disclosure refers to an antibody or antibody fragment specific for IL-17C wherein said antibody or antibody fragment binds to a region on human IL-17C, wherein the region comprises the amino acids PVLRPEEVL (SEQ ID NO.: 27) of human IL-17C. In another embodiment the present disclosure refers to an antibody or antibody fragment specific for IL-17C wherein said antibody or antibody fragment binds to a region on human IL-17C, wherein the region comprises the amino acids VLRPEEVL (SEQ ID NO.: 28) of human IL-17C.

In another embodiment the present disclosure refers to an antibody or antibody fragment specific for IL-17C wherein said antibody or antibody fragment binds to the region of amino acids aa 89-97 of SEQ ID No.: 1. In a further embodiment said antibody or antibody fragment binds to the region of amino acids aa 90-97 of SEQ ID No.: 1.

In one embodiment, said antibody or antibody fragment specific for IL-17C blocks the binding of IL-17C to the receptor of IL-17C. In a further embodiment, said antibody or antibody fragment specific for IL-17C blocks the binding of IL-17C to the receptor of IL17, wherein said receptor is IL17RE. In another embodiment the present disclosure refers to an antibody or antibody fragment specific for IL-17C, wherein said antibody or antibody fragment blocks the binding of IL-17C to IL17RE. In another embodiment said antibody or antibody fragment is an IL-17C antagonist. In a further embodiment said antibody or antibody fragment binds to an epitope on human IL-17C, wherein the epitope comprises one or more amino acid residues within the amino acids PVLRPEEVL (SEQ ID NO.: 27) of human IL-17C.

In certain embodiments, said antibody or antibody fragment specific for IL-17C blocks the binding of IL-17C to one or more receptors of IL-17C. In alternative embodiments, said antibody or antibody fragment specific for the receptor of IL-17C blocks the binding of IL-17C to receptors of IL-17C, wherein the receptors of IL17 include IL17RE and IL17RA. In alternative embodiments, said antibody or antibody fragment specific for the receptor of IL-17C blocks the binding of IL-17C to IL17RE and IL17RA.

In certain embodiments, said antibody or antibody fragment specific for IL-17C blocks the binding of IL-17C to IL17RE with an IC₅₀ concentration of less than 100nM, 90nM, 80nM, 70nM, 60nM, 50nM, 40nM, 30nM, 20nM, 10nM, 9nM, 8nM, 7nM, 6nM, 5nM, 4nM, 3nM, 2nM, 1nM, 100pM, 90pM, 80pM, 70pM, 60pM, 50pM, 40pM, 30pM, 20pM, 10pM, 9pM, 8pM, 7pM, 6pM, 5pM, 4pM, 3pM, 2pM or 1pM. In certain aspects the IC₅₀ concentration can be determined by ELISA; SET, FACS or MSD (Meso Scale Discovery).

In one embodiment the disclosed antibody or antibody fragment is specific for human IL-17C. In a further embodiment the disclosed antibody or antibody fragment specific for IL-17C is cross-reactive with IL-17C of another species, such as IL-17C from mouse, rat, rhesus monkey and/or cynomolgus monkey. In another embodiment the antibody or antibody fragment is specific for human IL-17C, cynomolgus monkey IL-17C and mouse IL-17C.

In one embodiment the disclosed antibody or antibody fragment is specific for human IL-17C encoded by the amino acid sequence of SEQ ID No.: 1. In one embodiment the disclosed antibody or antibody fragment is specific for a polypeptide comprising the amino acid sequence of SEQ ID No.: 1. In a further embodiment said monoclonal antibody or antibody fragment is a monoclonal antibody specific for a polypeptide consisting of the amino acid sequence of SEQ ID No.: 1. In another embodiment the disclosed antibody or antibody fragment is specific for human IL-17C encoded by the amino acid sequence of SEQ ID No.: 1 and is a monoclonal antibody or antibody fragment.

In one embodiment the disclosed antibody or antibody fragment specific for IL-17C is a monoclonal antibody or antibody fragment.

In one embodiment the disclosed antibody or antibody fragment specific for IL-17C is a human, humanized or chimeric antibody. In certain embodiments, said antibody or antibody fragment specific for IL-17C is an isolated antibody or antibody fragment. In another embodiment said antibody or antibody fragment is a recombinant antibody or antibody fragment. In a further embodiment said antibody or antibody fragment is a recombinant human antibody or antibody fragment. In a further embodiment said recombinant human antibody or antibody fragment is an isolated recombinant human antibody or antibody fragment. In a further embodiment said recombinant human antibody or antibody fragment or isolated recombinant human antibody or antibody fragment is monoclonal.

In another embodiment the present disclosure refers to an antibody or antibody fragment specific for human IL-17C wherein said antibody or antibody fragment bivalently binds to an IL-17C homodimer and forms a complex consisting of said antibody or antibody fragment and one IL-17C homodimer and wherein said antibody or antibody fragment blocks the binding of IL-17C to IL17RE and wherein said antibody or antibody fragment is a monoclonal antibody or antibody fragment.

In another embodiment the present disclosure refers to an antibody or antibody fragment specific for human IL-17C wherein said antibody or antibody fragment bivalently binds to an IL-17C homodimer and forms a complex consisting of said antibody or antibody fragment and one IL-17C homodimer and wherein said antibody or antibody fragment blocks the binding of IL-17C to IL17RE and wherein said antibody or antibody fragment is a monoclonal antibody or antibody fragment and wherein said antibody or antibody fragment is a human, humanized or chimeric antibody or antibody fragment.

In one embodiment the disclosed antibody or antibody fragment comprises a human heavy chain constant region and a human light chain constant region.

In one embodiment the disclosed antibody or antibody fragment is of the IgG isotype. In another embodiment said antibody is IgG1.

In one embodiment said antibody fragment is a bivalent antibody fragment.

In one embodiment, the present disclosure refers to an antibody or antibody fragment specific for IL-17C wherein said an antibody or antibody fragment forms a complex with an IL-17C homodimer and wherein said complex has a molecular weight lower than 200kDa. More specifically, said an antibody or antibody fragment forms a complex with an IL-17C homodimer, wherein at least 50% of said formed complexes have a molecular weight lower than 200kDa. In further embodiments, said an antibody or antibody fragment forms a complex with an IL-17C homodimer, wherein at least 50%, at least 60%, at least 70%, at least 80%, at least 90% or at least 95%, of said formed complexes have a molecular weight lower than 200kDa. In a further embodiment said antibody or antibody fragment binds to an epitope on human IL-17C, wherein the epitope comprises one or more amino acid residues within the amino acids PVLRPEEVL (SEQ ID NO.: 27) of human IL-17C

In one embodiment, the present disclosure refers to an antibody or antibody fragment comprising 6 CDRs defined by Kabat of any of the antibodies in Table 1. In another aspect, the disclosure pertains to an isolated monoclonal antibody or fragment thereof comprising 6 CDRs defined by Kabat of each of the antibodies in Table 1.

In certain embodiments, the present disclosure refers to antibodies or antibody fragments specific for IL-17C, wherein said antibodies or antibody fragments can bind to IL-17C with an affinity of about less than 100 nM, more preferably less than about 60 nM, and still more preferably less than about 30 nM. Further preferred are antibodies or antibody fragments that bind to IL-17C with an affinity of less than about 10 nM, and more preferably less than about 3 nM.

In certain embodiments, the present disclosure refers to antibodies or antibody fragments specific for IL-17C, wherein said antibodies or antibody fragments can bind to IL-17C with a monovalent affinity of about less than 100 nM, more preferably less than about 60 nM, and still more preferably less than about 30 nM. Further preferred are antibodies or antibody fragments that bind to IL-17C with a monovalent affinity of less than about 10 nM, and more preferably less than about 3 nM.

In another embodiment, the present disclosure refers to antibodies or antibody fragments specific for IL-17C, wherein said antibodies or antibody fragments have a bivalent affinity to IL-17C which is at least 2-fold, at least 5-fold, at least 10-fold, at least 100-fold, at least 1000-fold, at least 10000-fold, at least 100000-fold higher than its monovalent affinity to IL-17C. In a further embodiment the bivalent affinity of said antibodies or antibody fragments is determined in IgG-format, wherein the monovalent affinity of said antibodies or antibody fragments is determined in Fab-format.

In another embodiment, the present disclosure refers to antibodies or antibody fragments specific for IL-17C, wherein said antibodies or antibody fragments have a monovalent affinity to IL-17C with a dissociation rate constant (K_{D}) of less than 5×10⁻²M, less than 10⁻²M, less than 5×10⁻³M, less than 10⁻³M, less than 5×10⁻⁴M, less than 10⁻⁴M, less than 5×10⁻⁵M, less than 10⁻⁵M, less than 5×10⁻⁶M, less than 10⁻⁶M, less than 5×10⁻⁷M, less than 10⁻⁷M, less than 5×10⁻⁸M, less than 10⁻⁸M, less than 5×10⁻⁹M, less than 10⁻⁹M, less than 5×10⁻¹⁰M, less than 10⁻¹⁰M, less than 5×10⁻¹¹M, less than 10⁻¹¹M, less than 5×10⁻¹²M, less than 10⁻¹²M, less than 5×10⁻¹³M, less than 10⁻¹³M, less than 5×10⁻¹⁴M, less than 10⁻¹⁴M, less than 5×10⁻¹⁵M, or less than 10⁻¹⁵M and wherein said antibodies or antibody fragments in a bivalent format have an affinity to IL-17C with a dissociation rate constant (K_{D}) which is at least 2-fold, 5-fold, 10-fold, 100-fold, 1000-fold, 10000-fold, 100000-fold lower than the dissociation rate constant (KD) in a monovalent format. In a further embodiment the bivalent affinity of said antibodies or antibody fragments is determined in IgG-format, wherein the monovalent affinity of said antibodies or antibody fragments is determined in Fab-format.

In one embodiment the present disclosure refers to a nucleic acid composition comprising a nucleic acid sequence or a plurality of nucleic acid sequences encoding an antibody or antibody fragment that bivalently binds to an IL-17C homodimer and forms a complex consisting of said antibody or antibody fragment and one IL-17C homodimer. In another embodiment the present disclosure refers to a vector composition comprising a vector or a plurality of vectors comprising said nucleic acid sequence or said plurality of nucleic acid sequences encoding an antibody or antibody fragment that bivalently binds to an IL-17C homodimer and forms a complex consisting of said antibody or antibody fragment and one IL-17C homodimer. In another embodiment the present disclosure refers to a cell comprising said vector composition. In further embodiments said cell is a bacterial or mammalian cell.

In one embodiment the present disclosure refers to the use of an antibody or antibody fragment specific for IL-17C for the treatment of a disorder or condition associated with the undesired presence of IL-17C, wherein said antibody or antibody fragment bivalently binds to an IL-17C homodimer and forms a complex consisting of said antibody or antibody fragment and one IL-17C homodimer. In another embodiment said condition associated with the undesired presence of IL-17C is an inflammatory disorder or cancer.

In one embodiments, the present disclosure refers to the use of an antibody or antibody fragment specific for IL-17C for the treatment of an inflammatory disorder wherein said antibody or antibody fragment bivalently binds to an IL-17C homodimer and forms a complex consisting of said antibody or antibody fragment and one IL-17C homodimer. In other aspects, the present disclosure refers to the use of an antibody or antibody fragment specific for IL-17C in the preparation of a medicament for the treatment of an inflammatory disorder. In further embodiments, the present disclosure refers to a method for the treatment of an inflammatory disorder in a subject, comprising administering to the subject a pharmaceutical composition comprising an antibody or antibody fragment that bivalently binds to an IL-17C homodimer and forms a complex consisting of said antibody or antibody fragment and one IL-17C homodimer.

In one embodiments, the present disclosure refers to the use of an antibody or antibody fragment specific for IL-17C for the treatment of cancer, wherein said antibody or antibody fragment bivalently binds to an IL-17C homodimer and forms a complex consisting of said antibody or antibody fragment and one IL-17C homodimer. In other aspects, the present disclosure refers to the use of an antibody or antibody fragment specific for IL-17C in the preparation of a medicament for the treatment of cancer. In further embodiments, the present disclosure refers to a method for the treatment of cancer in a subject, comprising administering to the subject a pharmaceutical composition comprising an antibody or antibody fragment that bivalently binds to an IL-17C homodimer and forms a complex consisting of said antibody or antibody fragment and one IL-17C homodimer.

In one embodiment the present disclosure refers to a composition comprising an antibody or antibody fragment specific for IL-17C wherein said antibody or antibody fragment bivalently binds to an IL-17C homodimer and forms a complex consisting of said antibody or antibody fragment and one IL-17C homodimer and composition further comprising one or more pharmaceutically acceptable carriers and/or diluents. In one embodiment said composition is a pharmaceutical composition. In a further embodiment said composition is capable of antagonizing IL-17C in a subject in need thereof. In a further embodiment said composition is a pharmaceutical composition and capable of antagonizing IL-17C in a subject in need thereof.

In a further embodiment, said pharmaceutical composition further comprises one or more pharmaceutically acceptable carriers and/or diluents.

In another embodiment the present disclosure refers to the use of said pharmaceutical composition for the treatment of a disorder or condition associated with the undesired presence of IL-17C. In another embodiment said condition associated with the undesired presence of IL-17C is an inflammatory disorder or cancer.

The compositions of the present disclosure are preferably pharmaceutical compositions comprising an antibody or antibody fragment specific for IL-17C as disclosed herein and a pharmaceutically acceptable carrier, diluent or excipient, for the treatment of an inflammatory disorder or cancer.

In another embodiment, the present disclosure refers to a method for the prophylaxis of an inflammatory disorder in a subject, said method comprising administering an IL-17C antagonist to said subject. "Prophylaxis" as used in this context refers to methods which aim to prevent the onset of a disease or which delay the onset of a disease.

In further embodiments, the present disclosure refers to the use of a composition comprising an isolated antibody or antibody fragment specific for IL-17C for the treatment of an inflammatory disorder wherein said isolated antibody or antibody fragment bivalently binds to an IL-17C homodimer and forms a complex consisting of said isolated antibody or antibody fragment and one IL-17C homodimer. In other aspects, the present disclosure refers to the use of an antibody or antibody fragment specific for IL-17C in the preparation of a medicament for the treatment of an inflammatory disorder. In further embodiments, the present disclosure refers to a method for the treatment of an inflammatory disorder in a subject, comprising administering to the subject a pharmaceutical composition comprising an antibody or antibody fragment that bivalently binds to an IL-17C homodimer and forms a complex consisting of said isolated antibody or antibody fragment and one IL-17C homodimer.

In some embodiments said subject is a human. In alternative aspects said subject is a rodent, such as a rat or a mouse.

In another embodiment, the present disclosure refers to the use of a composition comprising an isolated antibody or antibody fragment specific for IL-17C for the treatment of cancer, wherein said isolated antibody or antibody fragment bivalently binds to an IL-17C homodimer and forms a complex consisting of said isolated antibody or antibody fragment and one IL-17C homodimer. In further embodiments, the present disclosure refers to an antibody or antibody fragment specific for IL-17C for use in the treatment of cancer. In another embodiments the present disclosure refers to the use of an antibody or antibody fragment specific for IL-17C in the preparation of a medicament for the treatment of cancer. In another embodiments, the present disclosure refers to a method for the treatment of cancer in a subject, comprising administering to the subject a pharmaceutical composition comprising an antibody or antibody fragment that bivalently binds to an IL-17C homodimer and forms a complex consisting of said antibody or antibody fragment and one IL-17C homodimer.

In some embodiments, the antibodies or antibody fragments specific for IL-17C of the present disclosure are administered subcutaneously. In other aspects the antibodies or antibody fragments specific for IL-17C of the present disclosure are administered intravenously, intra-articularly or intra-spinally.

Such carriers, diluents and excipients are well known in the art, and the skilled artisan will find a formulation and a route of administration best suited to treat a subject with the IL-17C antibodies or antibody fragments of the present disclosure.

In one embodiment, the disclosure pertains to an isolated monoclonal antibody or fragment thereof comprising a VH and a VL of any of the antibodies in Table 1.

In another embodiment, the disclosure refers to a nucleic acid encoding an isolated monoclonal antibody or fragment thereof wherein the nucleic acid comprises a VH and a VL of any of the antibodies in Table 1.

### Engineered and Modified Antibodies

An antibody or antibody fragment of the present disclosure can be a modified antibody or antibody fragment. In one embodiment of the present disclosure the modified antibody or antibody fragment is derived from the antibodies shown in Table 1. Thereby the antibodies shown in Table 1 can be used as starting material to engineer a modified antibody.

An antibody can be engineered by modifying one or more residues within one or both variable regions (i.e., VH and/or VL), for example within one or more CDR regions and/or within one or more framework regions. Additionally or alternatively, an antibody can be engineered by modifying residues within the constant region(s), for example to alter the effector function(s) of the antibody.

By engineering or modify an antibody improved variants of the parental clone can be achieved. Meanwhile various technologies e.g. to improve the affinity, to reduce immunogenicity and to increase the effector function of an antibody are established in the art.

Antibodies interact with target antigens predominantly through amino acid residues that are located in the six heavy and light chain complementarity determining regions (CDRs). Thus affinity maturation comprises the modification of specific CDRs to alter binding properties of an antibody. Affinity maturation includes site directed mutagenesis within the hypervariable regions and may comprise amino acid substitutions, additions or deletions. Another type of affinity maturation comprises the complete replacement of specific CDRs in a specific antibody with a library of respective CDRs. Modified antibodies thereupon can be analyzed in standard antigen binding assays (e.g. ELISA, FACS, BiaCore, SET analysis) for improved affinity to the respective antigen. (see, e.g., Riechmann et al., (1998) Nature 332:323-327; Jones et al., (1986) Nature 321:522-525; Queen et al., (1989) Proc. Natl. Acad., U.S.A. 86:10029-10033; U.S. Patent No. 5,225,539 to Winter, and U.S. Patent Nos. 5,530,101; 5,585,089; 5,693,762 and 6,180,370 to Queen et al.)

Because CDR sequences are responsible for most antibody-antigen interactions, it is possible to express recombinant antibodies that mimic the properties of specific naturally occurring antibodies by constructing expression vectors that include CDR sequences from the specific naturally occurring antibody grafted onto framework sequences from a different antibody with different properties. Such framework sequences can be obtained from public DNA databases or published references that include germline antibody gene sequences. For example, germline DNA sequences for human heavy and light chain variable region genes can be found in the "Vase" human germline sequence database (available on the Internet at www.mrc- cpe.cam.ac.uk/vbase), as well as in Kabat et al., (1991) Sequences of Proteins of Immunological Interest, Fifth Edition, U.S. Department of Health and Human Services, NIH Publication No. 91-3242; Chothia et al., (1987) J. Mol. Biol. 196:901-917; Chothia et al., (1989) Nature 342:877-883; and Al-Lazikani et al., (1997) J. Mol. Biol. 273:927-948; Tomlinson et al., (1992) J. fol. Biol. 227:776-798; and Cox et al., (1994) Eur. J Immunol. 24:827-836; the contents of each of which are expressly incorporated herein by reference.

### Bispecific Molecules and Multivalent Antibodies

In another aspect, the present disclosure features biparatopic, bispecific, multispecific or polyspecific molecules comprising an antigen binding site specific for IL-17C.

In another aspect the bispecific antigen binding molecule, is selected from the group consisiting of a bispecific-scFv, a tetravalent bispecific antibody, a cross-linked Fab or a bispecific IgG.

In another aspect, the present disclosure provides multivalent compounds comprising at least two antigen-binding sites derived from IL-17C specific antibodies. In another aspect, said antigen-binding sites can be linked together via protein fusion or covalent or non-covalent linkage.

Tetravalent compounds can be obtained for example by cross-linking antibodies of the antibodies of the disclosure with an antibody that binds to the constant regions of the antibodies of the disclosure, for example the Fc or hinge region. Trimerizing domain are described for example in Borean patent EP 1012280B1. Pentamerizing modules are described for example in PCT/EP97/05897.

An antibody of the disclosure, or the antigen-binding regions thereof, can be linked to another functional molecule, e.g., another peptide or protein (e.g., another antibody or ligand for a receptor) to generate a bispecific molecule that binds to at least two different binding sites or target molecules.

To create a bispecific molecule of the disclosure, an antibody can be functionally linked (e.g., by chemical coupling, genetic fusion, non-covalent association or otherwise) to one or more other binding molecules, such as another antibody, antibody fragment, peptide or binding mimetic, such that a bispecific molecule results.

The bispecific molecules of the present disclosure can be prepared by conjugating the constituent binding specificities, using methods known in the art. For example, each binding specificity of the bispecific molecule can be generated separately and then conjugated to one another. When the binding specificities are proteins or peptides, a variety of coupling or cross-linking agents can be used for covalent conjugation. Examples of cross-linking agents include protein A, carbodiimide, N-succinimidyl-S-acetyl-thioacetate (SATA), 5,5'-dithiobis (2-nitrobenzoic acid) (DTNB), o-phenylenedimaleimide (oPDM), N-succinimidyl-3-(2-pyridyldithio)propionate (SPDP), and sulfosuccinimidyl 4-(N-maleimidomethyl) cyclohaxane-I-carboxylate (sulfo-SMCC) (see e.g., Karpovsky et al., (1984) J. Exp. Med. 160:1686; Liu et al. (1985) Proc. Natl. Acad. Sci. USA 82:8648). Other methods include those described in Paulus (1985) Behring Ins. Mitt. No. 78:118-132; Brennan et al., (1985) Science 229:81-83), and Glennie et al., (1987) J. Immunol. 139: 2367-2375). Conjugating agents are SATA and sulfo-SMCC, both available from Pierce Chemical Co. (Rockford, IL).

When the binding specificities are antibodies, they can be conjugated by sulfhydryl bonding of the C-terminus hinge regions of the two heavy chains. In a particularly embodiment, the hinge region is modified to contain an odd number of sulfhydryl residues, for example one, prior to conjugation.

Alternatively, both binding specificities can be encoded in the same vector and expressed and assembled in the same host cell. This method is particularly useful where the bispecific molecule is a mAb x mAb, mAb x Fab, Fab x F (ab')2 or ligand x Fab fusion protein. A bispecific molecule of the disclosure can be a single chain molecule comprising one single chain antibody and a binding determinant, or a single chain bispecific molecule comprising two binding determinants. Bispecific molecules may comprise at least two single chain molecules. Methods for preparing bispecific molecules are described for example in U.S. Patent Number 5,260,203; U.S. Patent Number 5,455,030; U.S. Patent Number 4,881,175; U.S. Patent Number 5,132,405; U.S. Patent Number 5,091,513; U.S. Patent Number 5,476,786; U.S. Patent Number 5,013,653; U.S. Patent Number 5,258,498; and U.S. Patent Number 5,482,858.

Further clinical benefits may be provided by the binding of two or more antigens within one antibody (Morrison et al., (1997) Nature Biotech. 15:159-163; Alt et al. (1999) FEBS Letters 454: 90-94; Zuo et al., (2000) Protein Engineering 13:361-367; Lu et al., (2004) JBC 279:2856-2865; Lu et al., (2005) JBC 280:19665-19672; Marvin et al., (2005) Acta Pharmacologica Sinica 26:649-658; Marvin et al., (2006) Curr Opin Drug Disc Develop 9:184-193; Shen et al., (2007) J Immun Methods 218:65-74; Wu et al., (2007) Nat Biotechnol. 11:1290-1297; Dimasi et al., (2009) J Mol Biol. 393:672-692; and Michaelson et al., (2009) mAbs 1:128-141).

Binding of the bispecific or cross-reactive molecules to their specific targets can be confirmed by, for example, enzyme-linked immunosorbent assay (ELISA), radioimmunoassay (REA), FACS analysis, bioassay (e.g., growth inhibition), or Western Blot assay. Each of these assays generally detects the presence of antigen-antibody complexes of particular interest by employing a labeled reagent (e.g., an antibody) specific for the complex of interest.

### Scaffolds

Other antibody/immunoglobulin frameworks or scaffolds comprising "antigen-binding sites" can be employed in line with the present disclosure. This includes non-immunoglobulin based antibodies and scaffolds onto which CDRs of the disclosure can be grafted.

The fibronectin scaffolds are based on fibronectin type III domain (e.g., the tenth module of the fibronectin type III (10 Fn3 domain)). The fibronectin type III domain has 7 or 8 beta strands which are distributed between two beta sheets, which themselves pack against each other to form the core of the protein, and further containing loops (analogous to CDRs) which connect the beta strands to each other and are solvent exposed. There are at least three such loops at each edge of the beta sheet sandwich, where the edge is the boundary of the protein perpendicular to the direction of the beta strands (see US 6,818,418). These fibronectin-based scaffolds are not an immunoglobulin, although the overall fold is closely related to that of the smallest functional antibody fragment, the variable region of the heavy chain, which comprises the entire antigen recognition unit in camel and lama IgG. Because of this structure, the non-immunoglobulin antibody mimics antigen binding properties that are similar in nature and affinity to those of antibodies. These scaffolds can be used in a loop randomization and shuffling strategy in vitro that is similar to the process of affinity maturation of antibodies in vivo. These fibronectin-based molecules can be used as scaffolds where the loop regions of the molecule can be replaced with CDRs of the disclosure using standard cloning techniques.

Camelid antibody proteins obtained from members of the camel and dromedary (Camelus bactrianus and Calelus dromaderius) family including new world members such as llama species (Lama paccos, Lama glama and Lama vicugna) have been characterized with respect to size, structural complexity and antigenicity for human subjects. Certain IgG antibodies from this family of mammals as found in nature lack light chains, and are thus structurally distinct from the typical four chain quaternary structure having two heavy and two light chains, for antibodies from other animals. See WO1994/04678.

The ankyrin technology is based on using proteins with ankyrin derived repeat modules as scaffolds for bearing variable regions which can be used for binding to different targets. The ankyrin repeat module is a 33 amino acid polypeptide consisting of two anti-parallel α-helices and a β-turn. Binding of the variable regions is mostly optimized by using ribosome display.

Avimers are derived from natural A-domain. These domains are used by nature for protein-protein interactions and in human over 250 proteins are structurally based on A-domains. Avimers consist of a number of different "A-domain" monomers (2-10) linked via amino acid linkers. Avimers can be created that can bind to the target antigen using the methodology described in, for example, U.S. Patent Application Publication Nos. 20040175756; 20050053973; 20050048512; and 20060008844.

Affibody affinity ligands are small, simple proteins composed of a three-helix bundle based on the scaffold of one of the IgG-binding domains of Protein A. Protein A is a surface protein from the bacterium Staphylococcus aureus. This scaffold domain consists of 58 amino acids, 13 of which are randomized to generate affibody libraries with a large number of ligand variants (See e.g., US 5,831,012). Affibody molecules mimic antibodies; they have a molecular weight of 6 kDa, compared to the molecular weight of antibodies, which is 150 kDa. In spite of its small size, the binding site of affibody molecules is similar to that of an antibody.

Anticalins are products developed by the company Pieris ProteoLab AG. They are derived from lipocalins, a widespread group of small and robust proteins that are usually involved in the physiological transport or storage of chemically sensitive or insoluble compounds. Several natural lipocalins occur in human tissues or body liquids. The protein architecture is reminiscent of immunoglobulins, with hypervariable loops on top of a rigid framework. However, in contrast with antibodies or their recombinant fragments, lipocalins are composed of a single polypeptide chain with 160 to 180 amino acid residues, being just marginally bigger than a single immunoglobulin domain. The set of four loops, which makes up the binding pocket, shows pronounced structural plasticity and tolerates a variety of side chains. The binding site can thus be reshaped in a proprietary process in order to recognize prescribed target molecules of different shape with high affinity and specificity. One protein of lipocalin family, the bilin-binding protein (BBP) of Pieris Brassicae has been used to develop anticalins by mutagenizing the set of four loops. One example of a patent application describing anticalins is in PCT Publication No. WO1999/16873.

Affilin molecules are small non-immunoglobulin proteins which are designed for specific affinities towards proteins and small molecules. New affilin molecules can be very quickly selected from two libraries, each of which is based on a different human derived scaffold protein. Affilin molecules do not show any structural homology to immunoglobulin proteins. Currently, two affilin scaffolds are employed, one of which is gamma crystalline, a human structural eye lens protein and the other is "ubiquitin" superfamily proteins. Both human scaffolds are very small, show high temperature stability and are almost resistant to pH changes and denaturing agents. This high stability is mainly due to the expanded beta sheet structure of the proteins. Examples of gamma crystalline derived proteins are described in WO2001/04144 and examples of "ubiquitin-like" proteins are described in WO2004/106368.

Protein epitope mimetics (PEM) are medium-sized, cyclic, peptide-like molecules (MW 1-2kDa) mimicking beta-hairpin secondary structures of proteins, the major secondary structure involved in protein-protein interactions.

### Generation of Antibodies

### (i) Nucleic Acids Encoding the Antibodies

The disclosure provides substantially purified nucleic acid molecules which encode polypeptides comprising segments or domains of the antibody chains described above. In a specific embodiment, the nucleic acid molecules are those identified in Table 1. Some other nucleic acid molecules of the disclosure comprise nucleotide sequences that are substantially identical (e.g., at least 65, 80%, 85%, 90%, 95%, or 99%) to the nucleotide sequences of those identified in Table 1. Subcloning of above mentioned nucleic acids into conventional and appropriate expression vectors and expression of said expression vectors in an appropriate expression system originates polypeptides encoded by these polynucleotides which specifically bind to IL-17C.

Also provided in the disclosure are polynucleotides which encode at least one CDR region and usually all three CDR regions from the heavy or light chain of the antibody set forth above. Some other polynucleotides encode all or substantially all of the variable region sequence of the heavy chain and/or the light chain of the antibody set forth above. Because of the composition of the genetic code, a variety of nucleic acid sequences will encode each of the immunoglobulin amino acid sequences.

The polynucleotide sequences can be produced by de novo solid-phase DNA synthesis or by PCR mutagenesis of an existing sequence (e.g., sequences as described in the Examples below) encoding an antibody specific for IL-17C or its binding fragment. Direct chemical synthesis of nucleic acids can be accomplished by methods known in the art, such as the phosphotriester method of Narang et al., (1979) Meth. Enzymol. 68:90; the phosphodiester method of Brown et al., (1979) Meth. Enzymol. 68:109; the diethylphosphoramidite method of Beaucage et al., (1981) Tetra. Lett., 22:1859; and the solid support method of U.S. Patent No. 4,458,066. Introducing mutations to a polynucleotide sequence by PCR can be performed as described in, e.g., PCR Technology: Principles and Applications for DNA Amplification, H.A. Erlich (Ed.), Freeman Press, NY, NY, 1992; PCR Protocols: A Guide to Methods and Applications, Innis et al. (Ed.), Academic Press, San Diego, CA, 1990; Mattila et al., (1991) Nucleic Acids Res. 19:967; and Eckert et al., (1991) PCR Methods and Applications 1:17.

Also provided in the disclosure are expression vectors and host cells for producing the antibodies or antibody fragments described above. Various expression vectors can be employed to express the polynucleotides encoding the IL-17C specific antibody chains or binding fragments. Both viral-based and nonviral expression vectors can be used to produce the antibodies in a mammalian host cell. Nonviral vectors and systems include plasmids, episomal vectors, typically with an expression cassette for expressing a protein or RNA, and human artificial chromosomes (see, e.g., Harrington et al., (1997) Nat Genet 15:345). For example, nonviral vectors useful for expression of the polynucleotides and polypeptides in mammalian (e.g., human) cells include pThioHis A, B & C, pcDNA3.1/His, pEBVHis A, B & C, (Invitrogen, San Diego, CA), MPSV vectors, and numerous other vectors known in the art for expressing other proteins. Useful viral vectors include vectors based on retroviruses, adenoviruses, adenoassociated viruses, herpes viruses, vectors based on SV40, papilloma virus, HBP Epstein Barr virus, vaccinia virus vectors and Semliki Forest virus (SFV). See, Brent et al., (1995) Annu. Rev. Microbiol. 49:807; and Rosenfeld et al., (1992) Cell 68:143.

The choice of the expression vector depends on the intended host cells in which the vector is to be expressed. Typically, the expression vectors contain a promoter and other regulatory sequences (e.g., enhancers) that are operably linked to the polynucleotides encoding an antibody chain or fragment. In some embodiments, an inducible promoter is employed to prevent expression of inserted sequences except under inducing conditions. Inducible promoters include, e.g., arabinose, lacZ, metallothionein promoter or a heat shock promoter. Cultures of transformed organisms can be expanded under noninducing conditions without biasing the population for coding sequences whose expression products are better tolerated by the host cells. In addition to promoters, other regulatory elements may also be required or desired for efficient expression of an antibody chain or fragment. These elements typically include an ATG initiation codon and adjacent ribosome binding site or other sequences. In addition, the efficiency of expression may be enhanced by the inclusion of enhancers appropriate to the cell system in use (see, e.g., Scharf et al., (1994) Results Probl. Cell Differ. 20:125; and Bittner et al., (1987) Meth. Enzymol., 153:516). For example, the SV40 enhancer or CMV enhancer may be used to increase expression in mammalian host cells.

The expression vectors may also provide a secretion signal sequence position to form a fusion protein with polypeptides encoded by inserted antibody sequences. More often, the inserted antibody sequences are linked to signal sequences before inclusion in the vector. Vectors to be used to receive sequences encoding antibody light and heavy chain variable domains may also encode constant regions or parts thereof. Such vectors allow expression of the variable regions as fusion proteins with the constant regions thereby leading to production of intact antibodies or antibody fragments. Typically, such constant regions are human.

The host cells for harboring and expressing the antibody chains can be either prokaryotic or eukaryotic. *E. coli* is one prokaryotic host useful for cloning and expressing the polynucleotides of the present disclosure. Other microbial hosts suitable for use include bacilli, such as *Bacillus subtilis,* and other enterobacteriaceae, such as Salmonella, Serratia, and various Pseudomonas species. In these prokaryotic hosts, one can also make expression vectors, which typically contain expression control sequences compatible with the host cell (e.g., an origin of replication). In addition, any number of a variety of well-known promoters will be present, such as the lactose promoter system, a tryptophan (trp) promoter system, a beta-lactamase promoter system, or a promoter system from phage lambda. The promoters typically control expression, optionally with an operator sequence, and have ribosome binding site sequences and the like, for initiating and completing transcription and translation. Other microbes, such as yeast, can also be employed to express polypeptides of the disclosure. Insect cells in combination with baculovirus vectors can also be used.

In some preferred embodiments, mammalian host cells are used to express and produce the antibodies or antibody fragment specific for IL-17C of the present disclosure. For example, they can be a hybridoma cell line expressing endogenous immunoglobulin genes. Preferably a mammalian cell line is used harboring an exogenous expression vector including any normal mortal or normal or abnormal immortal animal or human cell. (e.g., the SP2/0 myeloma cells, CHO cells, HeLa cells, PER.C6 cells, COS cells, HKB11 cells, NS0 cells). For example, a number of suitable host cell lines capable of secreting intact immunoglobulins have been developed including the CHO cell lines, various Cos cell lines, HeLa cells, myeloma cell lines, transformed B-cells and hybridomas. The use of mammalian tissue cell culture to express polypeptides is discussed generally in, e.g., Winnacker, FROM GENES TO CLONES, VCH Publishers, N.Y., N.Y., 1987. Expression vectors for mammalian host cells can include expression control sequences, such as an origin of replication, a promoter, and an enhancer (see, e.g., Queen et al., (1986) Immunol. Rev. 89:49-68), and necessary processing information sites, such as ribosome binding sites, RNA splice sites, polyadenylation sites, and transcriptional terminator sequences. These expression vectors usually contain promoters derived from mammalian genes or from mammalian viruses. Suitable promoters may be constitutive, cell type-specific, stage-specific, and/or modulatable or regulatable. Useful promoters include, but are not limited to, the metallothionein promoter, the constitutive adenovirus major late promoter, the dexamethasone-inducible MMTV promoter, the SV40 promoter, the MRP pollll promoter, the constitutive MPSV promoter, the tetracycline-inducible CMV promoter (such as the human immediate-early CMV promoter), the constitutive CMV promoter, and promoter-enhancer combinations known in the art.

Methods for introducing expression vectors containing the polynucleotide sequences of interest vary depending on the type of cellular host. For example, calcium chloride transfection is commonly utilized for prokaryotic cells, whereas calcium phosphate treatment or electroporation may be used for other cellular hosts. (See generally Sambrook, et al., supra). Other methods include, e.g., electroporation, calcium phosphate treatment, liposome-mediated transformation, injection and microinjection, ballistic methods, virosomes, immunoliposomes, polycation nucleic acid conjugates, naked DNA, artificial virions, fusion to the herpes virus structural protein VP22 (Elliot and O'Hare, (1997) Cell 88:223), agent-enhanced uptake of DNA, and ex vivo transduction. For long-term, high-yield production of recombinant proteins, stable expression will often be desired.

### (ii) Generation of monoclonal Antibodies

Monoclonal antibodies (mAbs) can be produced by a variety of techniques, including conventional monoclonal antibody methodology e.g., the standard somatic cell hybridization technique of Kohler and Milstein, (1975) Nature 256: 495. Many techniques for producing monoclonal antibody can be employed e.g., viral or oncogenic transformation of B-lymphocytes.

An animal system for preparing hybridomas is the murine system. Hybridoma production in the mouse is a well-established procedure. Immunization protocols and techniques for isolation of immunized splenocytes for fusion are known in the art. Fusion partners (e.g., murine myeloma cells) and fusion procedures are also known.

Chimeric or humanized antibodies of the present disclosure can be prepared based on the sequence of a murine monoclonal antibody prepared as described above. DNA encoding the heavy and light chain immunoglobulins can be obtained from the murine hybridoma of interest and engineered to contain non-murine (e.g. human) immunoglobulin sequences using standard molecular biology techniques. For example, to create a chimeric antibody, the murine variable regions can be linked to human constant regions using methods known in the art (see e.g., U.S. Patent No. 4,816,567 to Cabilly et al.). To create a humanized antibody, the murine CDR regions can be inserted into a human framework using methods known in the art. See e.g., U.S. Patent No. 5225539 to Winter et al., and U.S. Patent Nos. 5530101; 5585089; 5693762 and 6180370 to Queen et al.

In a certain embodiment, the antibodies of the disclosure are human monoclonal antibodies. Such human monoclonal antibodies can be generated using transgenic or transchromosomic mice carrying parts of the human immune system rather than the mouse system. These transgenic and transchromosomic mice include mice referred to herein as HuMAb mice and KM mice, respectively, and are collectively referred to herein as "human Ig mice."

The HuMAb mouse^{®} (Medarex, Inc.) contains human immunoglobulin gene miniloci that encode un-rearranged human heavy (µ and γ) and κ light chain immunoglobulin sequences, together with targeted mutations that inactivate the endogenous µ and κ chain loci (see e.g., Lonberg, et al., (1994) Nature 368(6474): 856-859). Accordingly, the mice exhibit reduced expression of mouse IgM or κ, and in response to immunization, the introduced human heavy and light chain transgenes undergo class switching and somatic mutation to generate high affinity human IgGκ monoclonal (Lonberg et al. (1994) Handbook of Experimental Pharmacology 113:49-101; Lonberg and Huszar, (1995) Intern. Rev. Immunol.13: 65-93, and Harding and Lonberg, (1995) Ann. N. Y. Acad. Sci. 764:536-546). The preparation and use of HuMAb mice, and the genomic modifications carried by such mice, is further described in Taylor et al., (1992) Nucleic Acids Research 20:6287-6295; Chen et al., (1993) International Immunology 5: 647-656; Tuaillon et al., (1993) Proc. Natl. Acad. Sci. USA 94:3720-3724; Choi et al., (1993) Nature Genetics 4:117-123; Chen et al., (1993) EMBO J. 12:821-830; Tuaillon et al., (1994) J. Immunol. 152:2912-2920; Taylor et al., (1994) International Immunology 579-591; and Fishwild et al., (1996) Nature Biotechnology 14: 845-851, the contents of all of which are hereby specifically incorporated by reference in their entirety. See further, U.S. Patent Nos. 5,545,806; 5,569,825; 5,625,126; 5,633,425; 5,789,650; 5,877,397; 5,661,016; 5,814,318; 5,874,299; and 5,770,429; all to Lonberg and Kay; U.S. Patent No. 5,545,807 to Surani et al.; PCT Publication Nos. WO1992/103918, WO1993/12227, WO1994/25585, WO1997/113852, WO1998/24884 and WO1999/45962, all to Lonberg and Kay; and PCT Publication No. WO2001/14424.

In another embodiment, human antibodies of the disclosure can be raised using a mouse that carries human immunoglobulin sequences on transgenes and transchomosomes such as a mouse that carries a human heavy chain transgene and a human light chain transchromosome. Such mice, referred to herein as "KM mice", are described in detail in PCT Publication WO2002/43478 to Ishida et al.

Still further, alternative transgenic animal systems such as the Xenomouse (Abgenix, Inc.) can be used. Such mice are described in, e.g., U.S. Patent Nos. 5,939,598; 6,075,181; 6,114,598; 6, 150,584 and 6,162,963 to Kucherlapati et al.

Moreover, alternative transchromosomic animal systems expressing human immunoglobulin genes are available in the art and can be used to raise antibodies of the disclosure. For example, mice carrying both a human heavy chain transchromosome and a human light chain tranchromosome, referred to as "TC mice" can be used; such mice are described in Tomizuka et al., (2000) Proc. Natl. Acad. Sci. USA 97:722-727. Furthermore, cows carrying human heavy and light chain transchromosomes have been described in the art (Kuroiwa et al., (2002) Nature Biotechnology 20:889-894) and can be used to raise antibodies of the disclosure.

Preferably human monoclonal antibodies of the disclosure can also be prepared using phage display methods for screening libraries of human immunoglobulin genes. Such phage display methods for isolating human antibodies are established in the art or described in the examples below. See for example: U.S. Patent Nos. 5,223,409; 5,403,484; and 5,571,698 to Ladner et al.; U.S. Patent Nos. 5,427,908 and 5,580,717 to Dower et al.; U.S. Patent Nos. 5,969,108 and 6,172,197 to McCafferty et al.; and U.S. Patent Nos. 5,885,793; 6,521,404; 6,544,731; 6,555,313; 6,582,915 and 6,593,081 to Griffiths et al.

Human monoclonal antibodies of the disclosure can also be prepared using ribosome display, m-RNA display, bacterial display and yeast display methods for screening libraries of human immunoglobulin genes. In general eukaryotic cells displaying human antibody libraries are standard in the art (see Plückthun, A. (1997) Proc. Natl. Acad. Sci. U.S.A. 94 (10): 4937-42; Lipovsek et al. (2004) Imm. Methods 290 (1-2): 51-67; He et al. (2007) Nature 4 (3): 281-288; Gold et al. (2001) Proc Natl Acad Sci USA 98 (9): 4825-6; Fukuda I, Kojoh K, Tabata N, et al. (2006) Nucleic Acids Res. 34 (19): e127; Francisco et al. (1993) Proc. Nat. Acad. Sci. U.S.A. 90: 10444-48; Georgiou et al. (1997) Nat. Biotech. 15 (1): 29-34; Boder et al. (2000) Proc Nat Acad Sci, 97(20):10701-10705; Weaver-Feldhaus et al. (2004) FEBS Letters 564 (1-2): 24-34).

Human monoclonal antibodies of the disclosure can also be prepared using SCID mice into which human immune cells have been reconstituted such that a human antibody response can be generated upon immunization. Such mice are described in, for example, U.S. Patent Nos. 5,476,996 and 5,698,767 to Wilson et al.

### (iii) Framework or Fc engineering

One type of framework modification involves mutating one or more residues within the framework region, or even within one or more CDR regions, to remove T cell -epitopes to thereby reduce the potential immunogenicity of the antibody. This approach is also referred to as "deimmunization" and is described in further detail in U.S. Patent Publication No. 20030153043 by Carr et al.

In addition or alternative to modifications made within the framework or CDR regions, antibodies of the disclosure may be engineered to include modifications within the Fc region, typically to alter one or more functional properties of the antibody, such as serum half-life, complement fixation, Fc receptor binding, and/or antigen-dependent cellular cytotoxicity. Furthermore, an antibody of the disclosure may be chemically modified (e.g., one or more chemical moieties can be attached to the antibody) or be modified to alter its glycosylation, again to alter one or more functional properties of the antibody. Each of these embodiments is described in further detail below.

In one embodiment, the hinge region of CH1 is modified such that the number of cysteine residues in the hinge region is altered, e.g., increased or decreased. This approach is described further in U.S. Patent No. 5,677,425 by Bodmer et al. The number of cysteine residues in the hinge region of CH1 is altered to, for example, facilitate assembly of the light and heavy chains or to increase or decrease the stability of the antibody.

In another embodiment, the Fc hinge region of an antibody is mutated to decrease the biological half-life of the antibody. More specifically, one or more amino acid mutations are introduced into the CH2-CH3 domain interface region of the Fc-hinge fragment such that the antibody has impaired Staphylococcyl protein A (SpA) binding relative to native Fc-hinge domain SpA binding. This approach is described in further detail in U.S. Patent No. 6,165,745 by Ward et al.

In yet other embodiments, the Fc region is altered by replacing at least one amino acid residue with a different amino acid residue to alter the effector functions of the antibody. For example, one or more amino acids can be replaced with a different amino acid residue such that the antibody has an altered affinity for an effector ligand but retains the antigen-binding ability of the parent antibody. The effector ligand to which affinity is altered can be, for example, an Fc receptor or the C1 component of complement. The corresponding modified isotype version is known as IgG1f LALA in the scientific community. As already mentioned above, this approach is described in further detail in U.S. Patent Nos. 5,624,821 and 5,648,260, both by Winter et al.

In another embodiment, one or more amino acids selected from amino acid residues can be replaced with a different amino acid residue such that the antibody has altered C1q binding and/or reduced or abolished complement dependent cytotoxicity (CDC). This approach is described in further detail in U.S. Patent Nos. 6,194,551 by Idusogie et al.

In another embodiment, one or more amino acid residues are altered to thereby alter the ability of the antibody to fix complement. This approach is described further in PCT Publication WO1994/29351 by Bodmer et al.

In yet another embodiment, the Fc region is modified to increase the ability of the antibody to mediate antibody dependent cellular cytotoxicity (ADCC) and/or to increase the affinity of the antibody for an Fcγ receptor by modifying one or more amino acids. This approach is described further in PCT Publication WO2000/42072 by Presta. Moreover, the binding sites on human IgG1 for FcγRl, FcγRII, FcγRIII and FcRn have been mapped and variants with improved binding have been described (see Shields et al., (2001) J. Biol. Chem. 276:6591-6604).

In still another embodiment, the glycosylation of an antibody is modified. For example, an aglycoslated antibody can be made (i.e., the antibody lacks glycosylation). Glycosylation can be altered to, for example, increase the affinity of the antibody for "antigen'. Such carbohydrate modifications can be accomplished by, for example, altering one or more sites of glycosylation within the antibody sequence. For example, one or more amino acid substitutions can be made that result in elimination of one or more variable region framework glycosylation sites to thereby eliminate glycosylation at that site. Such aglycosylation may increase the affinity of the antibody for antigen. Such an approach is described in further detail in U.S. Patent Nos. 5,714,350 and 6,350,861 by Co et al.

Additionally or alternatively, an antibody can be made that has an altered type of glycosylation, such as a hypofucosylated antibody having reduced amounts of fucosyl residues or an antibody having increased bisecting GlcNac structures. Such altered glycosylation patterns have been demonstrated to increase the ADCC ability of antibodies. Such carbohydrate modifications can be accomplished by, for example, expressing the antibody in a host cell with altered glycosylation machinery. Cells with altered glycosylation machinery have been described in the art and can be used as host cells in which to express recombinant antibodies of the disclosure to thereby produce an antibody with altered glycosylation. For example, EP 1,176,195 by Hang et al. describes a cell line with a functionally disrupted FUT8 gene, which encodes a fucosyl-transferase, such that antibodies expressed in such a cell line exhibit hypofucosylation. PCT Publication WO2003/035835 by Presta describes a variant CHO cell line, Lecl3 cells, with reduced ability to attach fucose to Asn(297)-linked carbohydrates, also resulting in hypofucosylation of antibodies expressed in that host cell (see also Shields et al., (2002) J. Biol. Chem. 277:26733-26740). PCT Publication WO1999/54342 by Umana et al. describes cell lines engineered to express glycoprotein-modifying glycosyl-transferases (e.g., beta (1,4)-N acetylglucosaminyl-transferase III (GnTIII)) such that antibodies expressed in the engineered cell lines exhibit increased bisecting GlcNac structures which results in increased ADCC activity of the antibodies (see also Umana et al., (1999) Nat. Biotech. 17:176-180).

In another embodiment, the antibody is modified to increase its biological half-life. Various approaches are possible. For example, one or more of the following mutations can be introduced: T252L, T254S, and T256F, as described in U.S. Patent No. 6,277,375 to Ward. Alternatively, to increase the biological half-life, the antibody can be altered within the CH1 or CL region to contain a salvage receptor binding epitope taken from two loops of a CH2 domain of an Fc region of an IgG, as described in U.S. Patent Nos. 5,869,046 and 6,121,022 by Presta et al.

### Pharmaceutical Compositions

In some aspects, the present disclosure refers to a composition comprising an isolated antibody or antibody fragment specific for IL-17C wherein said isolated antibody or antibody fragment bivalently binds to an IL-17C homodimer and forms a complex consisting of said isolated antibody or antibody fragment and one IL-17C homodimer. In further aspects said composition is a pharmaceutical composition. In further aspects said pharmaceutical composition is used for the treatment of a disease.

A pharmaceutical composition of the present disclosure can be administered by a variety of methods known in the art. The route and/or mode of administration vary depending upon the desired results. It is preferred that administration be intravenous, intramuscular, intraperitoneal, or subcutaneous, or administered proximal to the site of the target. The pharmaceutically acceptable carrier should be suitable for intravenous, intramuscular, subcutaneous, parenteral, spinal or epidermal administration (e.g., by injection or infusion). Depending on the route of administration, the active compound, i.e. antibody, antibody fragment, bispecific and multispecific molecule, may be coated in a material to protect the compound from the action of acids and other natural conditions that may inactivate the compound.

Pharmaceutically carriers enhance or stabilize the composition, or to facilitate preparation of the composition. Pharmaceutically acceptable carriers include solvents, dispersion media, coatings, antibacterial and antifungal agents, isotonic and absorption delaying agents, and the like that are physiologically compatible.

The composition should be sterile and fluid. Proper fluidity can be maintained, for example, by use of coating such as lecithin, by maintenance of required particle size in the case of dispersion and by use of surfactants. In many cases, it is preferable to include isotonic agents, for example, sugars, polyalcohols such as mannitol or sorbitol, and sodium chloride in the composition. Long-term absorption of the injectable compositions can be brought about by including in the composition an agent which delays absorption, for example, aluminum monostearate or gelatin.

Pharmaceutical compositions of the disclosure can be prepared in accordance with methods well known and routinely practiced in the art. See, e.g., Remington: The Science and Practice of Pharmacy, Mack Publishing Co., 20th ed., 2000; and Sustained and Controlled Release Drug Delivery Systems, J.R. Robinson, ed., Marcel Dekker, Inc., New York, 1978. Pharmaceutical compositions are preferably manufactured under GMP conditions. Typically, a therapeutically effective dose or efficacious dose of the IL-17C antibody or antibody fragment is employed in the pharmaceutical compositions of the disclosure. Antibodies or antibody fragments are formulated into pharmaceutically acceptable dosage forms by conventional methods known to those of skill in the art. Dosage regimens are adjusted to provide the optimum desired response (e.g., a therapeutic response). For example, a single bolus may be administered, several divided doses may be administered over time or the dose may be proportionally reduced or increased as indicated by the exigencies of the therapeutic situation. It is especially advantageous to formulate parental compositions in dosage unit form for ease of administration and uniformity of dosage. Dosage unit form as used herein refers to physically discrete units suited as unitary dosages for the subjects to be treated; each unit contains a predetermined quantity of active compound calculated to produce the desired therapeutic effect in association with the required pharmaceutical carrier.

Actual dosage levels of the active ingredients in the pharmaceutical compositions of the present disclosure can be varied so as to obtain an amount of the active ingredient which is effective to achieve the desired therapeutic response for a particular patient, composition, and mode of administration, without being toxic to the patient. The selected dosage level depends upon a variety of pharmacokinetic factors including the activity of the particular compositions of the present disclosure employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors.

A physician or veterinarian can start doses of the antibodies of the disclosure employed in the pharmaceutical composition at levels lower than that required to achieve the desired therapeutic effect and gradually increase the dosage until the desired effect is achieved. In general, effective doses of the compositions of the present disclosure, for the treatment of an allergic inflammatory disorder described herein vary depending upon many different factors, including means of administration, target site, physiological state of the patient, whether the patient is human or an animal, other medications administered, and whether treatment is prophylactic or therapeutic. Treatment dosages need to be titrated to optimize safety and efficacy. For systemic administration with an antibody, the dosage ranges from about 0.0001 to 100 mg/kg, and more usually 0.01 to 15 mg/kg, of the host body weight. An exemplary treatment regime entails systemic administration once per every two weeks or once a month or once every 3 to 6 months. For intravitreal administration with an antibody, the dosage ranges from about 0.0001 to about 10 mg. An exemplary treatment regime entails systemic administration once per every two weeks or once a month or once every 3 to 6 months.

The antibodies or antibody fragments of the present disclosure are usually administered on multiple occasions. Intervals between single dosages can be weekly, monthly or yearly. In some methods of systemic administration, dosage is adjusted to achieve a plasma antibody concentration of 1-1000 µg/ml and in some methods 25-500 µg/ml.

Alternatively, the antibodies or antibody fragments can be administered as a sustained release formulation, in which case less frequent administration is required. Dosage and frequency vary depending on the half-life of the antigen-binding moiety in the patient. In general, human and humanized antibodies show longer half-life than that of chimeric antibodies and nonhuman antibodies. The dosage and frequency of administration can vary depending on whether the treatment is prophylactic or therapeutic. In prophylactic applications, a relatively low dosage is administered at relatively infrequent intervals over a long period of time. Some patients continue to receive treatment for the rest of their lives. In therapeutic applications, a relatively high dosage at relatively short intervals is sometimes required until progression of the disease is reduced or terminated, and preferably until the patient shows partial or complete amelioration of symptoms of disease. Thereafter, the patient can be administered a prophylactic regime.

To prepare pharmaceutical or sterile compositions including antibodies or antibody fragments, the antibody or antibody fragment is mixed with a pharmaceutically acceptable carrier or excipient.

The desired dose of antibodies or antibody fragments is about the same as for an antibody or polypeptide, on a moles/kg body weight basis. The desired plasma concentration of the antibodies or antibody fragments is about, on a moles/kg body weight basis. The dose may be at least 15 µg at least 20 µg, at least 25 µg, at least 30 µg, at least 35 µg, at least 40 µg, at least 45 µg, at least 50 µg, at least 55 µg, at least 60 µg, at least 65 µg, at least 70 µg, at least 75 µg, at least 80 µg, at least 85 µg, at least 90 µg, at least 95 µg, or at least 100 µg. The doses administered to a subject may number at least 1, 2, 3, 4, 5, 6, 7, 8, 9, 10, 11, or 12, or more.

For antibodies or antibody fragments of the disclosure, the dosage administered to a patient may be 0.0001 mg/kg to 100 mg/kg of the patient's body weight. The dosage may be between 0.0001 mg/kg and 20 mg/kg, 0.0001 mg/kg and 10 mg/kg, 0.0001 mg/kg and 5 mg/kg, 0.0001 and 2 mg/kg, 0.0001 and 1 mg/kg, 0.0001 mg/kg and 0.75 mg/kg, 0.0001 mg/kg and 0.5 mg/kg, 0.0001 mg/kg to 0.25 mg/kg, 0.0001 to 0.15 mg/kg, 0.0001 to 0.10 mg/kg, 0.001 to 0.5 mg/kg, 0.01 to 0.25 mg/kg or 0.01 to 0.10 mg/kg of the patient's body weight.

The dosage of the antibodies or antibody fragments of the disclosure may be calculated using the patient's weight in kilograms (kg) multiplied by the dose to be administered in mg/kg.

The dosage of the antibodies or antibody fragments of the disclosure may be 150 µg/kg or less, 125 µg/kg or less, 100 µg/kg or less, 95 µg/kg or less, 90 µg/kg or less, 85 µg/kg or less, 80 µg/kg or less, 75 µg/kg or less, 70 µg/kg or less, 65 µg/kg or less, 60 µg/kg or less, 55 µg/kg or less, 50 µg/kg or less, 45 µg/kg or less, 40 µg/kg or less, 35 µg/kg or less, 30 µg/kg or less, 25 µg/kg or less, 20 µg/kg or less, 15 µg/kg or less, 10 µg/kg or less, 5 µg/kg or less, 2.5 µg/kg or less, 2 µg/kg or less, 1.5 µg/kg or less, 1 µg/kg or less, 0.5 µg/kg or less, or 0.5 µg/kg or less of a patient's body weight.

Unit dose of the antibodies or antibody fragments of the disclosure may be 0.1 mg to 20 mg, 0.1 mg to 15 mg, 0.1 mg to 12 mg, 0.1 mg to 10 mg, 0.1 mg to 8 mg, 0.1 mg to 7 mg, 0.1 mg to 5 mg, 0.1 to 2.5 mg, 0.25 mg to 20 mg, 0.25 to 15 mg, 0.25 to 12 mg, 0.25 to 10 mg, 0.25 to 8 mg, 0.25 mg to 7 mg, 0.25 mg to 5 mg, 0.5 mg to 2.5 mg, 1 mg to 20 mg, 1 mg to 15 mg, 1 mg to 12 mg, 1 mg to 10 mg, 1 mg to 8 mg, 1 mg to 7 mg, 1 mg to 5 mg, or 1 mg to 2.5 mg.

Doses of antibodies or antibody fragments of the disclosure may be repeated and the administrations may be separated by at least 1 day, 2 days, 3 days, 5 days, 10 days, 15 days, 30 days, 45 days, 2 months, 75 days, 3 months, or at least 6 months.

An effective amount for a particular patient may vary depending on factors such as the condition being treated, the overall health of the patient, the method route and dose of administration and the severity of side effects (see, e.g., Maynard, et al. (1996) A Handbook of SOPs for Good Clinical Practice, Interpharm Press, Boca Raton, Fla.; Dent (2001) Good Laboratory and Good Clinical Practice, London, UK).

The route of administration may be by, e.g., topical or cutaneous application, injection or infusion by intravenous, intraperitoneal, intracerebral, intramuscular, intraocular, intraarterial, intracerebrospinal, intralesional, or by sustained release systems or an implant (see, e.g., Sidman et al. (1983) Biopolymers 22:547-556; Langer, et al. (1981) J. Biomed. Mater. Res. 15:167-277; Langer (1982) Chem. Tech. 12:98-105; Epstein, et al. (1985) Proc. Natl. Acad. Sci. USA 82:3688-3692; Hwang, et al. (1980) Proc. Natl. Acad. Sci. USA 77:4030-4034; U.S. Pat. Nos. 6,350,466 and 6,316,024). Where necessary, the composition may also include a solubilizing agent and a local anesthetic such as lidocaine to ease pain at the site of the injection. In addition, pulmonary administration can also be employed, e.g., by use of an inhaler or nebulizer, and formulation with an aerosolizing agent. See, e.g., U.S. Pat. Nos. 6,019,968, 5,985,320, 5,985,309, 5,934,272, 5,874,064, 5,855,913, 5,290,540, and 4,880,078; and PCT Publication Nos. WO1992/19244, WO1997/32572, WO1997/44013, WO1998/31346, and WO1999/66903, each of which is incorporated herein by reference their entirety.

A composition of the present disclosure may also be administered via one or more routes of administration using one or more of a variety of methods known in the art. As will be appreciated by the skilled artisan, the route and/or mode of administration will vary depending upon the desired results. Selected routes of administration for antibodies or antibody fragments of the disclosure include intravenous, intramuscular, intradermal, intraperitoneal, subcutaneous, spinal or other parenteral routes of administration, for example by injection or infusion. Parenteral administration may represent modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, a composition of the disclosure can be administered via a non-parenteral route, such as a topical, epidermal or mucosal route of administration, for example, intranasally, orally, vaginally, rectally, sublingually or topically.

Additional therapies (e.g., prophylactic or therapeutic agents), which can be administered in combination with the antibodies or antibody fragments of the disclosure may be administered less than 5 minutes apart, less than 30 minutes apart, 1 hour apart, at about 1 hour apart, at about 1 to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, at about 12 hours to 18 hours apart, 18 hours to 24 hours apart, 24 hours to 36 hours apart, 36 hours to 48 hours apart, 48 hours to 52 hours apart, 52 hours to 60 hours apart, 60 hours to 72 hours apart, 72 hours to 84 hours apart, 84 hours to 96 hours apart, or 96 hours to 120 hours apart from the antibodies or antibody fragments of the disclosure. The two or more therapies may be administered within one same patient visit.

The antibodies or antibody fragments of the disclosure and the other therapies may be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time, optionally, followed by the administration of a third therapy (e.g., prophylactic or therapeutic agent) for a period of time and so forth, and repeating this sequential administration, i.e., the cycle in order to reduce the development of resistance to one of the therapies, to avoid or reduce the side effects of one of the therapies, and/or to improve the efficacy of the therapies.

In certain embodiments, the antibodies or antibody fragments of the disclosure can be formulated to ensure proper distribution in vivo. For example, the blood-brain barrier (BBB) excludes many highly hydrophilic compounds. To ensure that the therapeutic compounds of the disclosure cross the BBB (if desired), they can be formulated, for example, in liposomes. For methods of manufacturing liposomes, see, e.g., U.S. Pat. Nos. 4,522,811; 5,374,548; and 5,399,331. The liposomes may comprise one or more moieties which are selectively transported into specific cells or organs, thus enhance targeted drug delivery (see, e.g., V. V. Ranade (1989) J. Clin. Pharmacol. 29:685). Exemplary targeting moieties include folate or biotin (see, e.g., U.S. Pat. No. 5,416,016 to Low et al); mannosides (Umezawa et al., (1988) Biochem. Biophys. Res. Commun. 153:1038); antibodies (P. G. Bloeman et al. (1995) FEBS Lett. 357:140; M. Owais et al. (1995) Antimicrob. Agents Chemother. 39:180); surfactant protein A receptor (Briscoe et al. (1995) Am. J. Physiol. 1233:134); p 120 (Schreier et al (1994) J. Biol. Chem. 269:9090); see also K. Keinanen; M. L. Laukkanen (1994) FEBS Lett. 346:123; J. J. Killion; I. J. Fidler (1994) Immunomethods 4:273.

The disclosure provides protocols for the administration of pharmaceutical composition comprising antibodies or antibody fragments of the disclosure alone or in combination with other therapies to a subject in need thereof. The therapies (e.g., prophylactic or therapeutic agents) of the combination therapies of the present disclosure can be administered concomitantly or sequentially to a subject. The therapy (e.g., prophylactic or therapeutic agents) of the combination therapies of the present disclosure can also be cyclically administered. Cycling therapy involves the administration of a first therapy (e.g., a first prophylactic or therapeutic agent) for a period of time, followed by the administration of a second therapy (e.g., a second prophylactic or therapeutic agent) for a period of time and repeating this sequential administration, i.e., the cycle, in order to reduce the development of resistance to one of the therapies (e.g., agents) to avoid or reduce the side effects of one of the therapies (e.g., agents), and/or to improve, the efficacy of the therapies.

The therapies (e.g., prophylactic or therapeutic agents) of the combination therapies of the disclosure can be administered to a subject concurrently. The term "concurrently" is not limited to the administration of therapies (e.g., prophylactic or therapeutic agents) at exactly the same time, but rather it is meant that a pharmaceutical composition comprising antibodies or antibody fragments of the disclosure are administered to a subject in a sequence and within a time interval such that the antibodies of the disclosure can act together with the other therapy(ies) to provide an increased benefit than if they were administered otherwise. For example, each therapy may be administered to a subject at the same time or sequentially in any order at different points in time; however, if not administered at the same time, they should be administered sufficiently close in time so as to provide the desired therapeutic or prophylactic effect. Each therapy can be administered to a subject separately, in any appropriate form and by any suitable route. In various embodiments, the therapies (e.g., prophylactic or therapeutic agents) are administered to a subject less than 15 minutes, less than 30 minutes, less than 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, 24 hours apart, 48 hours apart, 72 hours apart, or 1 week apart. In other embodiments, two or more therapies (e.g., prophylactic or therapeutic agents) are administered to a within the same patient visit.

The prophylactic or therapeutic agents of the combination therapies can be administered to a subject in the same pharmaceutical composition. Alternatively, the prophylactic or therapeutic agents of the combination therapies can be administered concurrently to a subject in separate pharmaceutical compositions. The prophylactic or therapeutic agents may be administered to a subject by the same or different routes of administration.

**Table 1: Antibody sequences**

| **Antibody#** | | **SEQ ID No.:** | **[aa] / DNA** |
|---|---|---|---|
| **mab_1** | HCDR1 | SEQ ID No.: 7 | FTFSSYAMS |
| | HCDR2 | SEQ ID No.: 8 | VSAISGSGGSTYYADSVKG |
| | HCDR3 | SEQ ID No.: 9 | GFRGGFFAFDV |
| | LCDR1 | SEQ ID No.: 10 | SGDKLGDKYAY |
| | LCDR2 | SEQ ID No.: 11 | LVIYQDSKRPS |
| | LCDR3 | SEQ ID No.: 12 | QAWTFPLLTW |
| | VL | SEQ ID No.: 13 | SYELTQPPSVSVSPGQTASITCSGDKLGDKYAYWYQ QKPGQSPVLVIYQDSKRPSGIPERFSGSNSGNTATLT ISGTQAEDEADYYCQAWTFPLLTWVFGGGTKLTVLG QPKAAPSVTLFPPSSEELQANKATLVCLISDFYPGAVT VAWKADSSPVKAGVETTTPSKQSNNKYAASSYLSLT PEQWKSHRSYSCQVTHEGSTVEKTVAPTECS |
| | VH (lgG1) | SEQ ID No.: 14 | EVQLLESGGGLVQPGGSLRLSCAASGFTFSSYAMSW VRQAPGKGLEWVSAISGSGGSTYYADSVKGRFTISR DNSKNTLYLQMNSLRAEDTAVYYCARGFRGGFFAFD VWGQGTLVTVSSASTKGPSVFPLAPSSKSTSGGTAA LGCLVKDYFPEPVTVSWNSGALTSGVHTFPAVLQSS GLYSLSSVVTVPSSSLGTQTYICNVNHKPSNTKVDKR VEPKSCDKTHTCPPCPAPELLGGPSVFLFPPKPKDTL MISRTPEVTCVVVDVSHEDPEVKFNWYVDGVEVHNA KTKPREEQYNSTYRVVSVLTVLHQDWLNGKEYKCKV SNKALPAPIEKTISKAKGQPREPQVYTLPPSREEMTK NQVSLTCLVKGFYPSDIAVEWESNGQPENNYKTTPP VLDSDGSFFLYSKLTVDKSRWQQGNVFSCSVMHEAL HNHYTQKSLSLSPGK |
| | VL (DNA) | SEQ ID No.: 15 | agctatgaactgacccagccgccgagcgttagcgttagcccaggccaga ccgccagcattacctgtagcggcgacaaactgggcgacaaatacgccta ctggtatcagcagaaaccgggccagagcccggtgctggttatctatcagg atagcaaacgcccgagcggcattccagaacgctttagcggcagcaacag cggcaacaccgccaccctgaccattagcggcacccaggccgaagacga agccgattattactgccaggcttggactttcccgctgctgacttgggtgtttggc ggcggtaccaagctgaccgtgctgggccagcccaaagccgcccctagcg tgaccctgttccccccaagcagcgaggaactccaggccaacaaggccac cctcgtgtgcctgatcagcgacttctaccctggcgccgtgaccgtggcctgg aaggccgatagcagccctgtgaaggccggcgtggaaaccaccaccccc agcaagcagagcaacaacaaatacgccgccagcagctacctgagcctg acccccgagcagtggaagtcccacagatcctacagctgccaggtcacac acgagggcagcaccgtggaaaagaccgtggcccccaccgagtgcagc |
| | VH (DNA) | SEQ ID No.: 16 | gaagtgcagctgctggaaagcggtggcggtctggtgcagccaggtggtag cctgcgcctgagctgtgccgcaagcggctttacctttagcagctatgccatg agctgggtgcgccaagcaccaggcaaaggcctggaatgggtgagcgcc attagcggcagcggtggcagcacctattatgccgatagcgtgaaaggtcg ctttaccattagtcgcgataacagcaaaaacaccctgtatctgcaaatgaac agcctgcgggcagaagataccgcagtttattattgcgcgcgaggattccgt ggtggtttcttcgcattcgatgtgtggggtcagggcaccctggttactgtctcg agcgcgtcgaccaaaggccccagcgtgttccctctggcccccagcagca agagcacctctggcggaacagccgccctgggctgcctggtcaaggacta cttccccgagcccgtgaccgtgtcctggaactctggcgccctgaccagcgg cgtgcacacctttccagccgtgctccagagcagcggcctgtacagcctgag cagcgtcgtgaccgtgcccagcagcagcctgggcacccagacctacatct gcaacgtgaaccacaagcccagcaacacaaaggtggacaagcgggtg gaacccaagagctgcgacaagacccacacctgtcccccctgccctgccc |
| | | | ctgaactgctgggaggcccctccgtgttcctgttccccccaaagcctaagga caccctgatgatcagccggacccccgaagtgacctgcgtggtggtggacg tgtcccacgaggaccctgaagtgaagtttaattggtacgtggacggcgtgg aagtgcacaacgccaagaccaagcccagagaggaacagtacaacagc acctaccgggtggtgtccgtgctgaccgtgctgcaccaggactggctgaac ggcaaagagtacaagtgcaaggtgtccaacaaggccctgcctgccccca tcgagaaaaccatcagcaaggccaaaggccagccccgcgagccccag gtgtacacactgccccctagccgggaagagatgaccaagaaccaggtgt ccctgacctgcctcgtgaagggcttctaccccagcgacattgccgtggaat gggagagcaacggccagcccgagaacaactacaagaccaccccccct gtgctggacagcgacggctcattcttcctgtacagcaagctgaccgtggac aagagccggtggcagcagggcaacgtgttcagctgctccgtgatgcacg aggccctgcacaaccactacacccagaagtccctgagcctgagccccgg caag |
| **mab_8** | HCDR1 | SEQ ID No.: 17 | FTFSSYAMS |
| | HCDR2 | SEQ ID No.: 18 | VSAISGSGGSTYYADSVKG |
| | HCDR3 | SEQ ID No.: 19 | GFAIRYYGFDY |
| | LCDR1 | SEQ ID No.: 20 | SGDKLGDKYAY |
| | LCDR2 | SEQ ID No.: 21 | LVIYQDSKRPS |
| | LCDR3 | SEQ ID No.: 22 | QVFTFPLVTT |
| | VL | SEQ ID No.: 23 | SYELTQPPSVSVSPGQTASITCSGDKLGDKYAYWYQ QKPGQSPVLVIYQDSKRPSGIPERFSGSNSGNTATLT ISGTQAEDEADYYCQVFTFPLVTTVFGGGTKLTVLGQ PKAAPSVTLFPPSSEELQANKATLVCLISDFYPGAVTV AWKADSSPVKAGVETTTPSKQSNNKYAASSYLSLTP EQWKSHRSYSCQVTHEGSTVEKTVAPTECS |
| | VH (lgG1) | SEQ ID No.: 24 | EVQLLESGGGLVQPGGSLRLSCAASGFTFSSYAMSW VRQAPGKGLEWVSAISGSGGSTYYADSVKGRFTISR DNSKNTLYLQMNSLRAEDTAVYYCARGFAIRYYGFD YWGQGTLVTVSSASTKGPSVFPLAPSSKSTSGGTAA LGCLVKDYFPEPVTVSWNSGALTSGVHTFPAVLQSS GLYSLSSVVTVPSSSLGTQTYICNVNHKPSNTKVDKR VEPKSCDKTHTCPPCPAPELLGGPSVFLFPPKPKDTL MISRTPEVTCVVVDVSHEDPEVKFNWYVDGVEVHNA KTKPREEQYNSTYRVVSVLTVLHQDWLNGKEYKCKV SNKALPAPIEKTISKAKGQPREPQVYTLPPSREEMTK NQVSLTCLVKGFYPSDIAVEWESNGQPENNYKTTPP |
| | | | VLDSDGSFFLYSKLTVDKSRWQQGNVFSCSVMHEAL HNHYTQKSLSLSPGK |
| | VL (DNA) | SEQ ID No.: 25 | agctatgaactgacccagccgccgagcgttagcgttagcccaggccaga ccgccagcattacctgtagcggcgacaaactgggcgacaaatacgccta ctggtatcagcagaaaccgggccagagcccggtgctggttatctatcagg atagcaaacgcccgagcggcattccagaacgctttagcggcagcaacag cggcaacaccgccaccctgaccattagcggcacccaggccgaagacga agccgattattactgccaggttttcactttcccgctggttactactgtgtttggcg gcggtaccaagctgaccgtgctgggccagcccaaagccgcccctagcgt gaccctgttccccccaagcagcgaggaactccaggccaacaaggccac cctcgtgtgcctgatcagcgacttctaccctggcgccgtgaccgtggcctgg aaggccgatagcagccctgtgaaggccggcgtggaaaccaccaccccc agcaagcagagcaacaacaaatacgccgccagcagctacctgagcctg acccccgagcagtggaagtcccacagatcctacagctgccaggtcacac acgagggcagcaccgtggaaaagaccgtggcccccaccgagtgcagc |
| | VH (DNA) | SEQ ID No.: 26 | gaagtgcagctgctggaaagcggtggcggtctggtgcagccaggtggtag cctgcgcctgagctgtgccgcaagcggctttacctttagcagctatgccatg agctgggtgcgccaagcaccaggcaaaggcctggaatgggtgagcgcc attagcggcagcggtggcagcacctattatgccgatagcgtgaaaggtcg ctttaccattagtcgcgataacagcaaaaacaccctgtatctgcaaatgaac agcctgcgggcagaagataccgcagtttattattgcgcgcgtggtttcgcaa tccgttattatggatttgattattggggccagggcaccctggttactgtctcgag cgcgtcgaccaaaggccccagcgtgttccctctggcccccagcagcaag agcacctctggcggaacagccgccctgggctgcctggtcaaggactactt ccccgagcccgtgaccgtgtcctggaactctggcgccctgaccagcggcg tgcacacctttccagccgtgctccagagcagcggcctgtacagcctgagca gcgtcgtgaccgtgcccagcagcagcctgggcacccagacctacatctgc aacgtgaaccacaagcccagcaacacaaaggtggacaagcgggtgga acccaagagctgcgacaagacccacacctgtcccccctgccctgcccctg aactgctgggaggcccctccgtgttcctgttccccccaaagcctaaggaca ccctgatgatcagccggacccccgaagtgacctgcgtggtggtggacgtgt cccacgaggaccctgaagtgaagtttaattggtacgtggacggcgtggaa gtgcacaacgccaagaccaagcccagagaggaacagtacaacagcac ctaccgggtggtgtccgtgctgaccgtgctgcaccaggactggctgaacgg caaagagtacaagtgcaaggtgtccaacaaggccctgcctgcccccatc gagaaaaccatcagcaaggccaaaggccagccccgcgagccccaggt gtacacactgccccctagccgggaagagatgaccaagaaccaggtgtcc ctgacctgcctcgtgaagggcttctaccccagcgacattgccgtggaatgg gagagcaacggccagcccgagaacaactacaagaccaccccccctgtg ctggacagcgacggctcattcttcctgtacagcaagctgaccgtggacaag agccggtggcagcagggcaacgtgttcagctgctccgtgatgcacgaggc cctgcacaaccactacacccagaagtccctgagcctgagccccggcaag |

### Working Examples

### Example 1: Antigen generation

Amino acid sequences of IL-17C from human, cynomolgus monkey and mouse, were aligned.

Without leader sequence, homology of 79% is shared among all three species.

**Table 2: Species sequence homologies**

| | Human | Cynomolgus | Mouse |
|---|---|---|---|
| Human | 100% | 95% | 79% |
| Cynomolgus | | 100% | 79% |
| Mouse | | | 100% |

### Antigen Production and Quality control:

IL-17C form different species was purchased from different providers or produced in-house and solubilised, if necessary. Per 100 µg protein 4 µl biotinylation reagent of the ECLTM biotinylation module were added and incubated for 60 min at room temperature in the dark with gentle agitation. Subsequently biotinylated protein was purified using ZebaTM Desalt spin columns and OD280nm was determined.

Antigens were biotinylated by using the ECLTM biotinylation module (GE Healthcare; #1061918). After biotinlyation the product was purified using ZebaTM Desalt spin columns (Pierce; #89889).

Biotinylated and non-biotinylated mouse, cynomolgus and human IL-17C were subjected to a quality control comprising analyses under denaturing, reducing and denaturing, non-reducing conditions in SDS-PAGE and in native state by High Pressure-Size Exclusion Chromatography (HP-SEC) and Dynamic Light Scattering (DLS).

HP-SEC was performed on a Dionex UltiMate 3000 Titanium HPLC system (Dionex Corporation, Germering, Germany) in combination with Wyatt miniDAWN Treos and Wyatt Optilab rEX (Wyatt Technology Europe, Dernbach, Germany). For separation a Tosoh TSK-Gel G3000SWxl column was used (Tosoh Bioscience, Stuttgart, Germany). For each sample 15 µg of protein was loaded onto the column, separation was performed at a flow rate of 0.5 ml/min and recorded analyzing the UV absorption at 280 nm. The running buffer was composed of 49 mM NaH₂PO₄, 51 mM Na₂HPO₄, 100 mM K₂SO₄, 0.0005% Tween-80 at pH 6.8.

All DLS experiments were performed using a DynaPro Titan cuvette system (Wyatt Technology Europe, Dernbach, Germany) with protein concentrations between 0.2 and 1.0 mg/ml. In case of precipitation or particle formation, the sample was centrifuged with 10.000 g for 5 minutes prior to the experiment.

### Cloning and Production of mouse and human IL-17 Receptor E/Fc

The extracellular domain (ECD) of mouse IL-17 receptor E (UniProt Q8BH06, isoform 1) and human IL-17 receptor E (UniProt Q8NFR9) were cloned in the expression vector pMAX_vk_Fc2_His using Kpnl and EcoRV resulting in C-terminal Fc2_H fusion constructs. Beside the natural leader (AG00158) a second construct with a Vκ-Leader was generated (AG00159).

Both constructs were transiently expressed in HKB11 cells. The cell suspension was scaled up three days post transfection and the cell culture supernatant was harvested 6 days post transfection. After sterile filtration, the solution was subjected to protein A affinity chromatography. Buffer exchange was performed to PBS and samples were sterile filtered (0.2 µm pore size). Protein concentrations were determined by UV-spectrophotometry. Purity of the products was analysed under denaturing, reducing and denaturing, non-reducing conditions in SDS-PAGE and in native state by HP-SEC and DLS.

### Example 2: Generation of Fab fragments and antibodies

For antibody generation the MorphoSys Ylanthia^{®} library was used to select Fab fragments against human IL-17C. The MorphoSys Ylanthia^{®} library (Tiller et al. mAbs 5:3, 1-26; May/June (2013) and U.S. Patent No. 8,728,981) is a commercially available phagemid library and employs the CysDisplay^{®} technology for displaying the Fab on the phage surface (Lohning et al., WO2001/05950).

### 1. Panning Strategy:

In order to select antibodies having specific properties such as the bivalent binding of one IL-17C homodimer only solution pannings were performed. The antigen in its natural homodimeric state can be presented best in solution and therefore panning on soluble antigen increases the likelihood to identify clones which bind bivalently to IL-17C.

Recombinant antibodies were generated from the MorphoSys Ylanthia^{®} library by three iterative rounds of panning. Therefore biotinylated human IL-17C or biotinylated mouse IL-17C were used as antigens and incubated with the phage library in solution. Prerequisite for a solution panning was biotinylation of the antigen with preservation of its bio-activity. During solution panning, the Fab displaying phage and the biotinylated antigen were incubated in solution which facilitated the accessibility of the antigen by the phage.

For each phage pool, streptavidin beads (Dynabeads^{®} M-280 Streptavidin; Invitrogen) were blocked in 1x Chemiblocker. In parallel, for each panning, Ylanthia^{®} phage-antibodies were blocked with an equal volume of 2x Chemiblocker/Tween20.

Then, 100 nM biotinylated antigen was added to the pre-adsorbed and blocked phage particles and incubated at room temperature. The phage-antigen complexes were captured using blocked Streptavidin beads and phage particles bound to the Streptavidin beads were collected with a magnetic separator. Unspecifically bound phage were washed off by several washing steps using PBS, Tween20 and PBS and specifically bound phage were eluted from Streptavidin beads using DDT. The DTT eluate was then transferred into *E. coli* TG1 bacteria and incubated at 37°C for phage infection. The bacterial pellets were resuspended in growth medium, plated on LB/Cam agar plates and incubated overnight. Colonies were scraped off the plates and were used for phage rescue, polyclonal amplification of selected clones, and phage production.

Subsequent panning round 2 and 3 were performed in a similar fashion with prolonged washing steps and reduced antigen concentration to increase stringency and discard antibodies having low specificity and affinity. The antigens were used either consistently throughout the 3 rounds of panning or in an alternating manner. The antigens used for carboxy bead panning rounds 1-3 within two different settings are indicated in Table 3.

**Table 3: Panning Strategies**

| | 1^{st} round | 2^{nd} round | 3^{rd} round |
|---|---|---|---|
| Setting 1 | Human IL-17C-bio [100nM] | Human IL-17C-bio [10nM] | Human IL-17C-bio [1nM] |
| Setting 2 | Human IL-17C-bio [100nM] | Mouse IL-17C-bio [50nM] | Human IL-17C-bio [10nM] |

After three rounds of panning, the output of the third round was used for creating single colonies and picking these Fab-producing colonies randomly into 384-well plates.

### 2. Primary screening of panning output via ELISA to identify clones specific for human, cynomolgus and mouse IL-17C

Specificity of the Fabs to IL-17C was investigated via primary ELISA screening. To facilitate rapid expression of soluble Fab fragments in crude bacterial lysates periplasmatic extracts were prepared as previously described (Rauchenberger et al. (2003) J. Biol. Chem. 278.40: 38194-205). Fab containing *E. coli* lysates were used for ELISA screening of the initial hits.

For ELISA screening on biotinylated antigen Maxisorp^{™} 384 well plates were coated with Fd fragment specific sheep anti-human IgG (Binding site, #PC075) diluted 1:1000 in PBS. After blocking with 5% skim milk powder in PBS, Ylanthia^{®}-Fab-containing *E. coli* lysates were added and captured by the anti-Fd antibody to the plate. After extensive washing with PBST 0.5µg/ml of biotinylated human IL-17C was added. After another washing procedure the captured Ylanthia^{®}-Fab fragments were allowed to bind the biotinylated human IL-17C, which was detected by incubation with streptavidin conjugated to alkaline phosphatase followed by addition of AttoPhos fluorescence substrate (Roche: #11681982001). Fluorescence emission at 535 nm was recorded with excitation at 430 nm. To exclude clones expressing Fab fragments with non-specific binding, a counter screening on an irrelevant antigen as negative control antigen was performed in parallel. ELISA signals greater than 5-fold over background on biotinylated human IL-17C and less than 2-fold over background on irrelevant antigen were considered as positive hits.

For further selection of clones that cross-reactively bind to cynomolgus and mouse IL-17C in addition to human IL-17C the identical screening on biotinylated cynomolgus and mouse IL-17C was performed. The clones identified to be cross-reactive also to mouse and cynomolgus IL-17C antigen, were combined and collected onto a separate compression plate before subjected to the screening in the receptor binding inhibition assay.

In total 4608 individual Fab fragments were picked and produced in *E.coli.* Therefrom 1491 hits were identified to specifically bind to human IL-17C in bacterial lysate preparations in an ELISA screening. The 1491 hits identified to specifically bind to human IL-17C were further tested for ELISA binding to biotinylated cynomolgus and mouse IL-17C antigens. Of these, 723 Fabs turned out to be cross-reactive to biotinylated cynomolgus and mouse IL-17C.

### 3. Secondary screening of IL-17C specific clones for antagonistic activity

In a next screening step, the inhibitory functionality of these Fabs was investigated. Therefore Fab containing bacterial extracts were screened for inhibitory activity in a high-throughput m/hulL17RE/Fc receptor inhibition assay.

To test Fab-containing crude bacterial lysates for neutralizing activity in high throughput screening mode, MA6000 384 well plates (Meso Scale Discovery, MSD) were coated with 30 µl mouse or human IL17RE/Fc chimeric protein at 0.5-0.6 µg/ml in PBS overnight at 4°C. The next day 20-25 µl Fab-containing *E. coli* lysates were pre-incubated for 30 min at RT with an equal volume of biotinylated mouse or human IL-17C at 1-2 nM to form Fab-antigen complexes. After blocking of plates for 1h with 5% BSA in PBS, the complexes were added to the wells coated with mouse or human IL17RE/Fc and receptor binding was detected via Streptavidin-ECL using MSD Sector Imager.

A signal reduction > 60 % in m/hulL-17RE inhibition assay was defined as positive hit. In total, receptor inhibition screening yielded 285 clones inhibiting binding of IL-17C to its respective receptor (human or mouse IL17RE/Fc).

### 4. Identification of unique clones

The clones, which were identified to block IL-17C binding to its respective human or mouse IL-17RE receptor were picked onto another compression plate and the heavy and light chain genes of respective candidates were sequenced in order to identify unique sequences among all 285 clones.

Of the sequenced clones a number of 17 unique clones could be identified. The most frequent heavy chain is the VH3-23 with 8 antibodies, 3 antibodies have a VH3-07 chain, 2 antibodies VH3-11 and VH1-46, 1 antibody each have a VH3-15 or VH3-21 heavy chain. The most frequent light chain is the Vlambda3-1 with 8 antibodies, 4 antibodies with a Vkappa1-12 chain, 2 antibodies with Vlambda2-23 and 1 antibody each have a Vkappa1-06, Vkappa1-51 or Vkappa3-15 light chain. Respective frameworks and heavy/light chains are provided in the Ylanthia^{®} antibody library as disclosed in US 13/321,564 or US 13/299,367, which both herein are incorporated by reference.

### 5. Cloning of Fab-encoding DNA into expression vector and expression/purification

All 17 unique Ylanthia^{®} antibodies were subcloned into the bacterial Fab expression vector. For expression and purification *E.coli* bacteria were transformed with the Fab-encoding expression vector. Cell culture supernatant was harvested after induction of expression and subjected to purification via His-tag specific affinity chromatography (GE Healthcare) for His-tagged Fab purification. All samples were sterile filtered (0.2 µm pore size). Purity of Fabs was analyzed under denaturing, reducing and non-reducing conditions using a Labchip System (Caliper GXII, Perkin Elmer) or on SDS-PAGE. Protein concentrations were determined by UV-spectrophotometry.

### 6. IgG conversion of Ylanthia^{®} antibodies and expression/purification

The 17 unique Ylanthia^{®} clones were converted from Fab to IgG format by subcloning procedure. All 17 antibody encoding vectors were enzymatically digested and the resulting vector backbones ligated with the Ylanthia^{®} mammalian expression cassette and further subcloned into the respective full-length IgG vector.

For expression and purification eukaryotic HKB11 cells were transfected with pYMex10 eukaryotic expression vector DNA encoding both heavy and light chains of IgGs. Cell culture supernatant was harvested on day 3 post transfection and subjected to standard Protein A affinity chromatography (MabSelect SURE, GE Healthcare) for antibody purification. All samples were sterile filtered (0.2 µm pore size). Purity of IgG was analyzed under denaturing, reducing and non-reducing conditions using a Labchip System (Caliper GXII, Perkin Elmer) or on SDS-PAGE. Protein concentrations were determined by UV-spectrophotometry and HP-SEC was performed to analyze IgG preparations in native state.

All 17 unique Ylanthia^{®} IgGs were produced in exploratory-scale in HKB11 cells in the human IgG1 isotype format. All 17 IgGs showed good expression yields (> 5 mg/L) and only 3/17 did not pass quality control analysis due to unwanted aggregation tendencies.

Altogether 14 individual antibodies could be produced in good quantities and successfully passed quality control in SEC (> 90% monomer content). All 14 purified IgG1 antibodies were tested for binding to IL-17C in ELISA and were subjected to further functional testi ng.

### 7. Identification of IL-17C specific antibodies bivalently binding to IL-17C homodimer via size exclusion chromatography.

As a soluble homodimer IL-17C constitutes of two IL-17C monomers each having a molecular weight of 21,765 Da. Therefore, the homodimeric IL-17C has a total molecular weight of ~44 kDa and can be bound by a monoclonal antibody basically in three different ways forming three different complexes **(****Figure 1****).**

Depending on the specificity of the antibody mainly one out of three complexes is formed, wherein either two homodimers are associated with one antibody (class I), two homodimers are associated with two antibodies (class II) or one homodimer is associated with one antibody (class III). Accordingly, the formed complexes differ in their molecular weight wherein Class I complexes have a molecular weight of -270 kDa, Class II complexes have a molecular weight of -380 kDa and Class III complexes have a molecular weight of ~190 kDa.

In order to select for antibodies which bivalently bind to one IL-17C homodimer and mainly form class III complexes with a molecular weight lower than 200kDa the antibodies were incubated with human IL-17C homodimer and were subjected to size exclusion chromatography.

Analysis of the binding mode was done by mixing equimolar amounts of antigen and respective antibodies. The complexes created by antigen-antibody interaction were analyzed on size-exclusion-chromatography (SEC) and the size of the respective complexes determined by MALS. Respective complexes formed were detected by a Light scattering detector.

The binding mode was extrapolated by comparison of the detected complexes to the theoretical molecular weight of 1:1 (-190 kDa), 1:2 (-270 kDa) and 2:2 (-380 kDa) complexes.

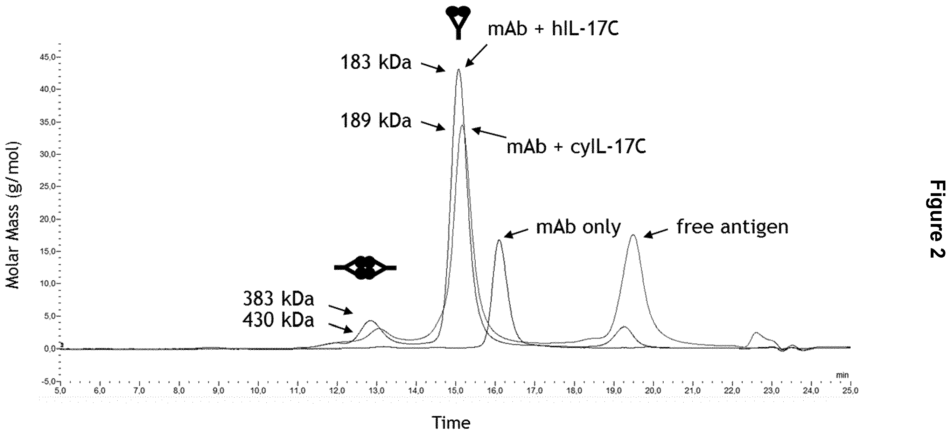

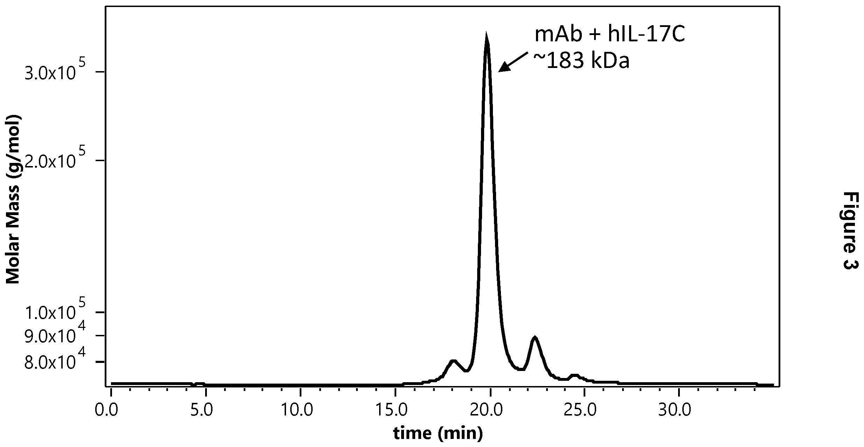

2 antibodies (mab_1, mab_8) were found to form mainly class III complexes and therefore bivalently bind to one IL-17C homodimer. (Figures 2, 3). Both antibodies have a VH3-23 heavy chain and a Vlambda3-1 light chain as provided in the Ylanthia^{®} antibody library as disclosed in US 13/321,564 or US 13/299,367, which both herein are incorporated by reference.

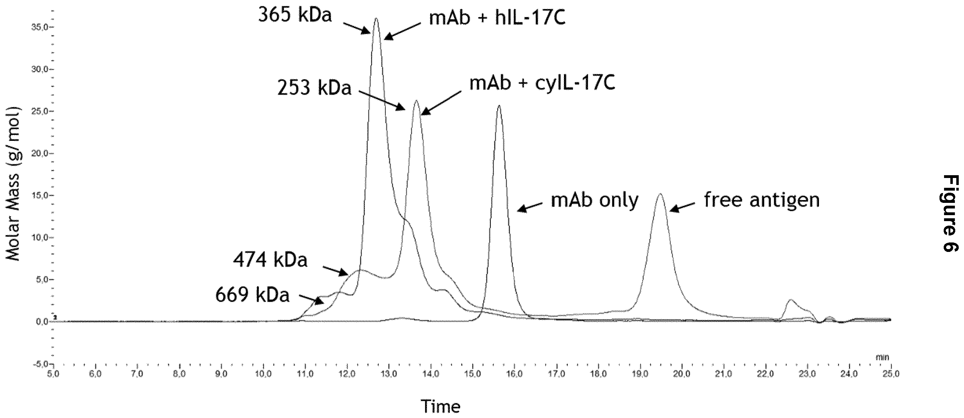

In contrast other antibodies which not bivalently bind to one IL-17C homodimer were identified to not form complexes with a molecular weight below 200kDa (Class III) but form larger complexes like class I and class II or others **(****Figure 6****).**

### Example 3: Characterization of IL-17C specific IgGs for receptor inhibition activity

Purified IL-17C specific IgGs were tested in mIL-17 RE interaction assay. Therefore MA6000 384 well plates (Meso Scale Discovery, MSD) were coated with 30 µl mouse IL17RE/Fc chimeric protein at 75 ng/ml in PBS at 4°C overnight. The next day a serial antibody dilution (concentrations from 0.001 to 100 nM) were pre-incubated for 30 min at RT with an equal volume of biotinylated IL-17C. After blocking of plates for 1h with 2.5% BSA in PBST, previously formed antibody-ligand complexes were added for 1h to coated IL17RE/Fc and receptor binding was detected via Streptavidin-ECL using MSD Sector Imager. Results are shown in Table 4.

**Table 4: Receptor Inhibition Assay IC₅₀ [nM]**

| **No.** | **Y-Number** | **Receptor binding inhibition IC₅₀ [nM]** |
|---|---|---|
| | | **hIL-17C-hIL17RE** |
| **1** | **mab_1** | **0,04** |
| 2 | mab_2 | 3,79 |
| 3 | mab_3 | 1,26 |
| 4 | mab_4 | - |
| 5 | mab_5 | 16,25 |
| 6 | mab_6 | 4,28 |
| 7 | mab_7 | 24,93 |
| **8** | **mab_8** | **0,13** |
| 9 | mab_9 | 0,82 |
| 10 | mab_10 | 22,43 |
| 11 | mab_11 | 0,05 |
| 12 | mab_12 | 61,53 |
| 13 | mab_13 | 17,86 |
| 14 | mab_14 | 15,92 |

### Example 4: Functional testing in IL-17C-driven NF-κB reporter assay

Purified IL-17C specific IgGs were further tested for their ability to inhibit the biological activity of human, mouse and cynomolgus IL-17C in a functional cell based assay that monitors the IL-17C driven activation of a NF-κB reporter gene in NIH3T3 cells overexpressing the murine IL-17RE.

NIH3T3 cells were cultured in DMEM supplemented with 10% FBS and 1% Pen/Strep at 37°C, 5% CO2. For the assay, NIH3T3 cells were transfected in suspension with total amount of 100ng DNA (20 ng mouse IL-17RE expression construct, 50ng NF-κB luciferase reporter construct and 30ng pBluescript) using the Polyplus jet-PEI transfection agent. In brief, the DNA was diluted in 5µ! 150 mM NaCl (per well) and 0.2µl jet-PEI in 8µ! 150 mM NaCl (per well) was prepared. After 5 minutes incubation at room temperature, the JetPEI solution was added to the DNA solution and further incubated for 20-30 minutes at room temperature. NIH3T3 cells were diluted to have -40,000 cells in 87µl medium. The cells were added to the DNA-JetPEI mix (87µl cells and 13µl DNA-JetPEI mix/well) and the final volume was transferred into 96 well plates.

After an overnight incubation at 37°C in a humidified 5% CO2 incubator, the medium was removed and replaced with 90µl medium containing 5% FBS and 1% Pen/Strep. 10µl of a serial antibody dilution made in DPBS that was pre-incubated for 30 minutes at room temperature with an equal volume of purified recombinant IL-17C (either human IL-17C (Novus #NBP1-42910), mouse mIL-17C (R&D Systems #2306-ml-025) or cynomolgus IL-17C (produced in house)), was added to the cells. The final concentration of IL-17C was 0.5 ng/ml. After incubating the plates at 37°C in CO2 incubator, 100 µl SteadyLite Plus (Perkin Elmer) is added followed by readout of the luminescence on the Envision (Perkin Elmer).

The two antibodies (mab_1, mab_8) were found to inhibit IL-17C mediated signaling most effectively as illustrated in Table 5.

**Table 5: Functional NF-kB reporter gene assay (IgG) IC₅₀ [pM]. NT = not tested**

| **No.** | **Y-Number** | **NF-kB reporter gene assay (IgG) IC₅₀ [pM]** | | |
|---|---|---|---|---|
| | | **hIL-17C** | **cyIL-17C** | **mIL-17C** |
| **1** | **mab_1** | **29 ±15** | **24 ±18** | **46 ±26** |
| 2 | mab_2 | 8256 | 9952 | 12772 ±6353 |
| 3 | mab_3 | 14051 ±1881 | 5261 ±2413 | 32756 |
| 4 | mab_4 | 1100 | 4700 | 9800 |
| 5 | mab_5 | 34533 | 26441 | 52295 |
| 6 | mab_6 | 7482 ±5236 | 24466 | *NT* |
| 7 | mab_7 | 8590 | 3618 | 3618 |
| **8** | **mab_8** | **42 ±49** | **39 ±51** | **1772 ±1712** |
| 9 | mab_9 | 1339 ±808 | 953 ±78 | 5922 ±4184 |
| 10 | mab_10 | *NT* | 26800 | *NT* |
| 11 | mab_11 | 1722 ±621 | 2985 ±668 | 11173 |
| 12 | mab_12 | 133333 | 44667 | *NT* |
| 13 | mab_13 | 6578 | 20622 ±19862 | 460 ±335 |
| 14 | mab_14 | 21580 | 31250 | 24796 |

### Example 5: Functional testing in IL-17C-driven DEF4B gene expression in primary human keratinocytes

In a further functional experiment IL-17C mediated beta-defensin 2 expression in human keratinocytes was analyzed in the presence and absence of mab_1 and mab_8.

NHEK (normal human epidermal keratinocytes) were obtained from Lonza and cultured in Keratinocyte Growth Medium with supplements (KGM-Gold^{™} Bullet kit, Lonza) following manufacturer's protocol. NHEKs that were subcultured to and cryopreserved at passage 3 were thawed and immediately seeded in KGM cell culture medium at 30,000 cells/well in a 96 well cell culture plate. After 2 days, the medium was removed and changed to KGM-Gold w/o hydrocortisone prior to addition of hIL-17C (Novus #NBP1-42910) and hTNFα (Peprotech #300-01A) to final concentrations of 10 ng/ml each.

For testing antibodies, the human IL-17C was first pre-incubated for 30 minutes at room temperature with an equal volume of a serial dilution of antibody made in DPBS. Cells were cultured for 48 hours and then total RNA was extracted using RNeasy 96 Kit (Qiagen), reverse transcribed using Taqman^{®} Reverse Transcription Reagents (Applied Biosystems) and expression of beta-defensin 2 (DEFB4A) was determined quantitative PCR (qPCR). In brief, 10µl PCR reactions were prepared using Taqman^{®} universal PCR master mix/No AmpErase^{®} UNG and predesigned Assay-on-demand Gene expression primer/probe sets for DEF4B (#Hs00823638_m1, all Applied Biosystems). qPCR was performed on the ViiA7^{™} Real-Time PCR instrument (Applied Biosystems). Gene expression was normalized to a housekeeping gene either β-actin (Taqman primer set #4310881E) or GAPDH (Taqman primer set #4310893E).

Both antibodies (mab_1, mab_8) were shown to effectively reduce beta-defensin 2 expression and confirmed their ability to neutralize the biological activity of human IL-17C (Table 6).

**Table 6: Functional keratinocyte assay (expression of DEFB4A) IC₅₀ [pM]**

| **No.** | **Y-Number** | **NHEK assay IC₅₀ [pM]** |
|---|---|---|
| | | **hIL-17C** |
| **1** | **mab_1** | **415±159** |
| **2** | **mab_8** | **93±54** |

### Example 6: Affinity determination in monovalent Fab and bivalent IgG format

Additionally monovalent Fab and bivalent IgG affinity was determined by SET. Therefore purified Fabs and IgGs were titrated on human or mouse IL-17C for EC₅₀ determination. The results confirmed the proposed avidity effect exerted by IgG antibodies forming mainly class III complexes.

Solution equilibrium titration (SET) was basically performed as described in the literature (Friquet et al., (1985) J. Immunol. Meth. 77: 305-19). In order to improve the sensitivity and accuracy of the SET method, it was transferred from classical ELISA to ECL based technology (Haenel et al. (2005) Anal Biochem. 339.1: 182-84).

As shown in Table 6 the determined EC₅₀ values of mab_1 and mab_8 IgG antibodies were significantly improved on human IL-17C and also on mouse IL-17C compared to the respective Fabs, indicating high avidity effects based on the bivalent binding to one IL-17C homodimer.

**Table 7: Affinity determination via SET in Fab and IgG, EC₅₀ [pM]**

| Y-/MOR-Number | Fab | | IgG | |
|---|---|---|---|---|
| | SET monovalent affinity EC₅₀ [pM] | | IgG bivalent affinity EC₅₀ [pM] | |
| | hGT454 | mGT454 | hGT454 | mGT454 |
| **mab_1** | **4800** | **72000** | **17** | **150** |
| mab_4 | 1100 | 9800 | 380 | 9200 |
| **mab_8** | **2700** | **490000** | **22** | **3350** |
| mab_9 | 8500 | 97000 | 730 | n.d. |
| mab_11 | 4500 | 50000 | 1500 | n.d. |

### Example 7: ELISA-based cross-competition assay

Cross-competition of an anti-IL-17C antibody or another IL-17C binding agent may be detected by using an ELISA assay according to the following standard procedure.

The general principle of the ELISA-assay involves coating of an anti-IL-17C antibody onto the wells of an ELISA plate. An excess amount of a second, potentially cross-competitive, anti-IL-17C antibody is then added in solution (i.e. not bound to the ELISA plate). Subsequently a limited amount of IL-17C-Fc is then added to the wells.

The antibody which is coated onto the wells and the antibody in solution will compete for binding of the limited number of IL-17C molecules. The plate is then washed to remove IL-17C molecules that has not bound to the coated antibody and to also remove the second, solution phase antibody as well as any complexes formed between the second, solution phase antibody and IL-17C. The amount of bound IL-17C is then measured using an appropriate IL-17C detection reagent. Therefore, IL-17C may be fused with a tag, e.g. Fc, Flag, etc. which can be detected via an appropriate tag-specific agent.

An antibody in solution that is cross-competitive to the coated antibody will be able to cause a decrease in the number of IL-17C molecules that the coated antibody can bind relative to the number of IL-17C molecules that the coated antibody can bind in the absence of the second, solution phase antibody.

This assay is described in more detail further below for two antibodies termed Ab-X and Ab-Y. In the instance where Ab-X is chosen to be the immobilized antibody, it is coated onto the wells of the ELISA plate, after which the plates are blocked with a suitable blocking solution to minimize non-specific binding of reagents that are subsequently added. An excess amount of Ab-Y is then added to the ELISA plate such that the moles of Ab-Y IL-17C binding sites per well are at least 10 fold higher than the moles of Ab-X IL-17C binding sites that are used, per well, during the coating of the ELISA plate. IL-17C is added such that the moles of IL-17C added per well were at least 25-fold lower than the moles of Ab-X IL-17C binding sites that are used for coating each well. Following a suitable incubation period, the ELISA plate is washed and a detection reagent specific for the IL-17C antigen is added to measure the amount of IL-17C molecules specifically bound by the coated anti-IL-17C antibody (in this case Ab-X). The background signal for the assay is defined as the signal obtained in wells with the coated antibody (in this case Ab-X), second solution phase antibody (in this case Ab-Y), buffer only (i.e. no IL-17C) and detection reagents. The positive control signal for the assay is defined as the signal obtained in wells with the coated antibody (in this case Ab-X), second solution phase antibody buffer only (i.e. no second solution phase antibody), IL-17C detection reagents. The ELISA assay needs to be run in such a manner so as to have the positive control signal be at least 6 times the background signal.

To avoid any artifacts (e.g. significantly different affinities between Ab-X and Ab-Y for IL-17C) resulting from the choice of which antibody to use as the coating antibody and which to use as the second (competitor) antibody, the cross-blocking assay needs to be run in two formats: 1) format 1 is where Ab-X is the antibody that is coated onto the ELISA plate and Ab-Y is the competitor antibody that is in solution and 2) format 2 is where Ab-Y is the antibody that is coated onto the ELISA plate and Ab-X is the competitor antibody that is in solution.

### Example 8: Epitope determination using Hydrogen/Deuterium Exchange Mass Spectrometry

HDX mass spectrometry (also HDX MS) makes use of the principle that labile protons of proteins exchange with protons of the solvent in aqueous solution. The reaction kinetics is dependent on the temperature, pH, acidic nature of the respective amino acids and the degree of exposure to the solvent of the proton in question. By replacing the solvent from hydrogen to deuterium based aqueous solutions, deuterium is rapidly incorporated in solvent exposed areas of the protein, replacing the respective hydrogen atoms. After digestion of the protein, the peptide masses are analyzed using mass spectrometry. The gain in mass upon incorporation of deuterium can be identified. By comparing the peptide masses of antigens with and without the bound antibody fragment, the epitope of the antibody can be determined.

Therefore, antigen samples, uncomplexed and complexed (with the respective antibody) are subjected to deuterium solvent for various incubation times (e.g. 0 sec, 10 sec, 30 sec, 2 min, 10 min, 30 min, 1 hour). At each given time point, the samples are rapidly digested by injection into a pepsin column and deuterium exchange conditions are quenched using low pH and low temperature to preserve the specific deuterium pattern. The resulting deuterated and undeuterated peptides are desalted and separated using reversed phase (RP) nano-UPLC separation and injected into a high-resolution Q-IMS-TOF mass spectrometer. Chromatographic conditions are optimized to maximize the peptide sequence coverage of the complete antigen sample.

To identify the epitope of an antibody, the kinetic rates of deuterium incorporation of each peptide is compared in the unbound and complexed state. Reduced incorporation rates of a specific peptide in the complexed state, results from the shielding of the peptide from the surrounding solvent by the bound antibody. For visualization purposes, the differential uptake of deuterium is plotted onto the sequence of the antigen.

### Liquid handling and chromatographic setup

Automated hydrogen-deuterium exchange mass spectrometry (HDX MS) experiments are designed based upon methods described by T.E. Wales and J.R. Engen (Mass Spectrom Rev. 2006 Jan-Feb;25(1):158-70). The analytical equipment used consists of HDX Manager, nano-UPLC chromatography system and high-resolution mass spectrometer supplied by Waters Corporation (Milford, MA, USA). In brief, liquid handling operations are carried out in a Waters automated HDX manager with refrigerated sample compartments maintained at 2°C. The HDX Manager comprises of a PAL HTS Liquid handler, two Waters M-class UPLC Pumpsystems and a refrigerated compartment for sample trapping and chromatographic separation. Deuterium exchange is performed by incubating the protein complex samples for various times using the liquid handling system. Subsequently, the samples are injected into a 6-port injection valve and digested online on a Waters Pepsin column at 20 °C. Proteolytic peptides are automatically collected on a Waters Trapping column and injected into a Waters C-18 reversed-phase column for separation. The desalted and separated proteolytic peptides are directly injected into the electrospray ionization (ESI) source of the Synapt G2si mass spectrometer.

### Mass spectrometric analysis

Proteolytic peptides are identified using a Waters Synapt G2si (Q-IMS-TOF) mass spectrometer. Ion mobility separation is used to gain further resolution in case of simultaneously eluting peaks.. In addition, higher resolution can be achieved using fragmentation MS/MS analysis. In this case fragmentation of the respective peptides is initialized using electron-transfer dissociation (ETD), to avoid loss of information by deuterium scrambling.

### Preparation of Protein and Protein:Fab Complexes

Protein:Fab complexes are prepared by mixing 50 µg IL-17C in a 1:1 molar ratio with the Fab's and incubated for at least 2 hours at 4°C.

For on-exchange experiments IL-17C or IL-17C:Fab complexes are diluted with D₂O buffer. To gain insight into the kinetics of deuterium incorporation samples are analyzed at various time points of incubation in the D₂O buffer (e.g. 0 sec, 10 sec, 30 sec, 2 min, 10 min, 30 min, and 1 hour). The mixture is reduced by adding a reduction buffer before quenching with a quench buffer. Once mixed, the quenched solution is injected into the chromatographic system where it is automatically digested, separated and analyzed by LC-MS. The average change in deuteration between the sample and the control is calculated as the difference between the deuterium uptake levels of the sample and the control.

### Data Processing

Mass spectrometric raw data is evaluated using Waters MassLynx, HDX Manager, BiopharmaLynx and Dynamix software packages. MassLynx and HDX Manager Software is used for recording the spectrometric data. Subsequently all proteolytic peptides are identified using BiopharmaLynx. This information is used to evaluate the amount of deuterium uptake for each individual peptide using Waters Dynamix software.

The level of deuterium uptake is reported as the difference in mass between a deuterated sample and non-deuterated reference peptide. Processed data is manually assessed and adjusted to correct inaccuracies and errors from automated processing steps. Deuterium uptake levels are assigned to each residue of the protein sequence by delocalizing the deuterium content across each peptide (i.e., dividing the observed deuteration level by the number of amino acids in that peptide).

If a residue was covered by more than one peptide, the normalized deuterium uptakes of all peptides covering that residue are averaged by the software. If possible additional information from MS/MS analysis are used to increase the resolution of the analysis.

### Results

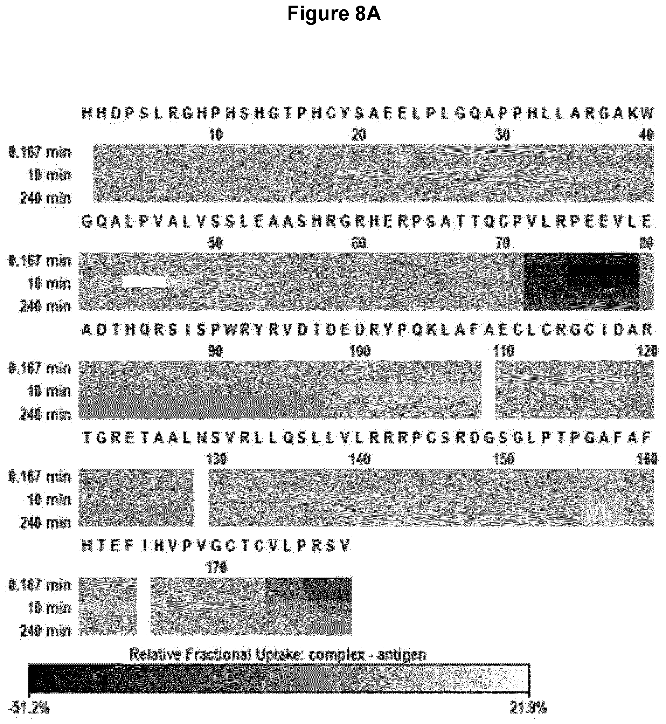

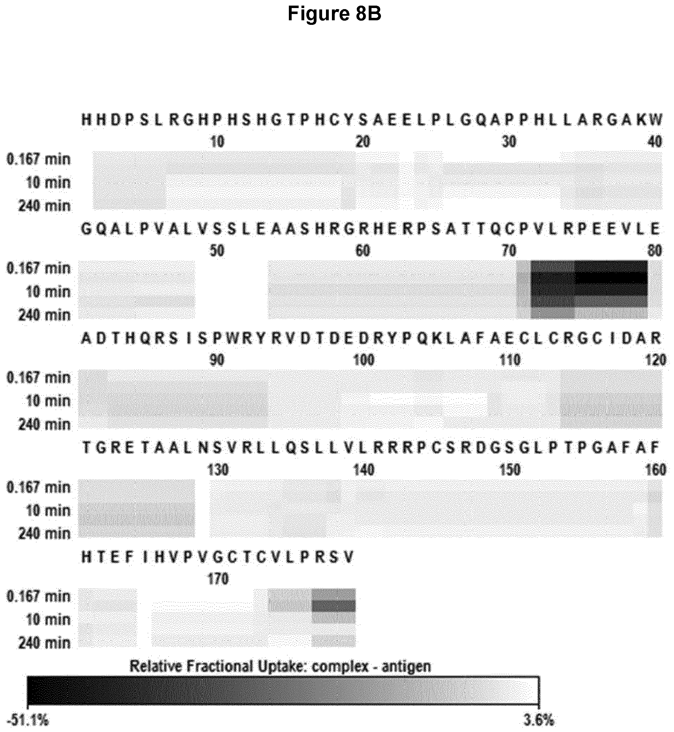

For the antibodies mab_1 and mab_8 the epitope was determined using hydrogen-deuterium exchange mass spectrometry (HDX MS) as outlined above. For both antibodies, mab_1 and mab_8, the same protection areas on human IL-17C were identified via HDX MS epitope mapping. The coverage maps **(****Figures 8A** **and** **8B****)** clearly identify the region PVLRPEEVL (SEQ ID No.: 27, aa 89-97 of SEQ ID No.: 1) as the main epitope on human IL-17C for both antibodies.

Three additional regions on human IL-17C (aa 98-111 (ADTHQRSISPWRY; SEQ ID No.: 29), aa 132-146 (CRGCIDARTGRETAAL; SEQ ID No.: 30) and aa 192-197 (TCVLPRSV; SEQ ID No.: 31) with very weak protection were also identified for both antibodies. However, due to the low level of protection these regions are not necessary part of the epitope.

The results indicate that the exemplified antibodies which bind to the region PVLRPEEVL (SEQ ID NO.: 27) on IL-17C show also bivalent binding of one IL-17C homodimer.

### Example 9: Complex determination of further antibodies by SEC

In addition further antibodies were analyzed for their binding characteristics according to the method as described in Example 2, Section 7.

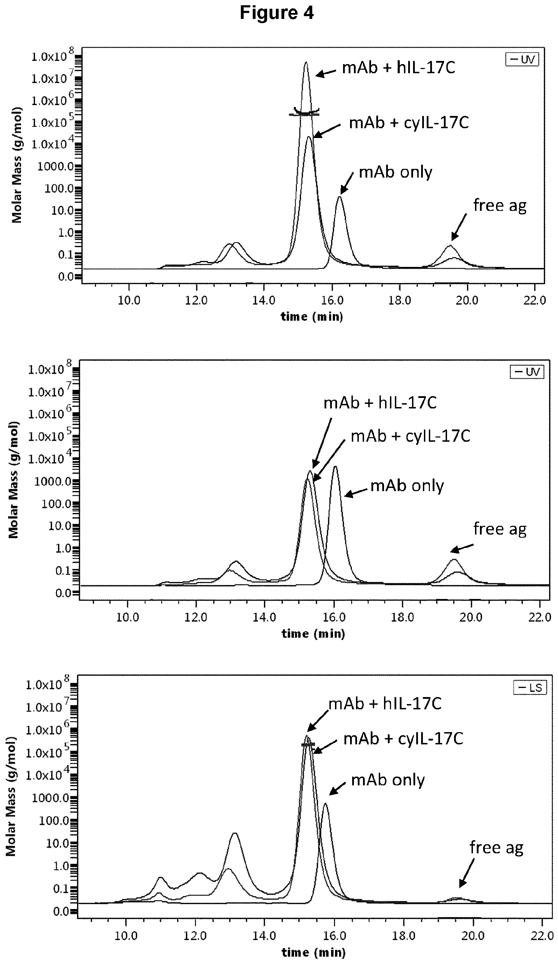

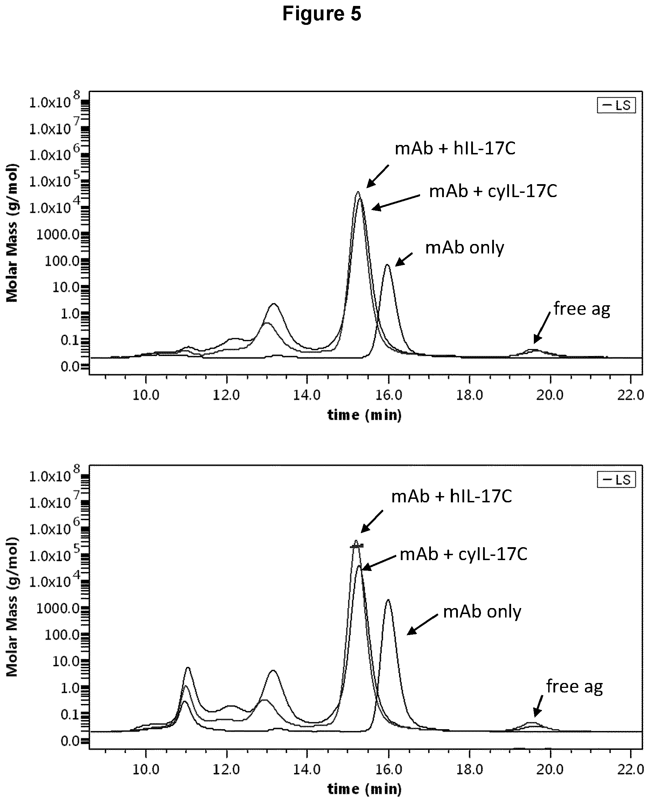

5 additional antibodies show bivalent binding of one IL-17C homodimer in Size Exclusion Chromatography (SEC). Results from the SEC for all 5 antibodies are exemplified in Figures 4-5.

In Figure 6 two antibodies are exemplified which do not bivalently bind to one IL-17C homodimer. Both antibodies do not form complexes with a molecular weight below 200kDa (Class III) but amongst others form larger complexes like class I and class II.

## Claims

1. An antibody or antibody fragment specific for human IL-17C wherein said antibody or antibody fragment bivalently binds to an IL-17C homodimer and forms a complex consisting of said antibody or antibody fragment and one IL-17C homodimer, and wherein the antibody or antibody fragment binds to the region PVLRPEEVL (SEQ ID NO.: 27) on IL-17C, and wherein said antibody or antibody fragment blocks the binding of IL-17C to IL17RE.

2. An antibody or antibody fragment according to any one of the preceding claims, wherein said antibody or antibody fragment is a monoclonal antibody or antibody fragment.

3. An antibody or antibody fragment according to any one of the preceding claims, wherein said antibody or antibody fragment is an isolated antibody or antibody fragment.

4. An antibody or antibody fragment according to any one of the preceding claims, wherein said antibody or antibody fragment is a recombinant antibody or antibody fragment.

5. An antibody or antibody fragment according to any one of the preceding claims for use in the treatment of an inflammatory disorder or cancer.

6. A nucleic acid composition comprising a nucleic acid sequence or a plurality of nucleic acid sequences encoding the antibody or antibody fragment according to any one of the preceding claims.

7. A vector composition comprising a vector or a plurality of vectors comprising the nucleic acid sequence or plurality of nucleic acid sequences of claim 7.

8. A cell comprising the vector composition of claim 8.

9. A pharmaceutical composition comprising an antibody or antibody fragment according to any one of the claims 1 to 6 and a pharmaceutically acceptable carrier or excipient.

## Patentansprüche

1. Antikörper oder Antikörperfragment, der bzw. das für menschliches IL-17C spezifisch ist, wobei der Antikörper bzw. das Antikörperfragment bivalent an ein IL-17C-Homodimer bindet und einen aus dem Antikörper bzw. Antikörperfragment und einem IL-17C-Homodimer bestehenden Komplex bildet und wobei der Antikörper bzw. das Antikörperfragment an die Region PVLRPEEVL (SEQ ID NO.: 27) auf IL-17C bindet und wobei der Antikörper bzw. das Antikörperfragment die Bindung von IL-17C an IL17RE blockiert.

2. Antikörper oder Antikörperfragment nach einem der vorhergehenden Ansprüche, wobei es sich bei dem Antikörper bzw. Antikörperfragment um einen monoklonalen Antikörper bzw. ein monoklonales Antikörperfragment handelt.

3. Antikörper oder Antikörperfragment nach einem der vorhergehenden Ansprüche, wobei es sich bei dem Antikörper bzw. Antikörperfragment um einen isolierten Antikörper bzw. ein isoliertes Antikörperfragment handelt.

4. Antikörper oder Antikörperfragment nach einem der vorhergehenden Ansprüche, wobei es sich bei dem Antikörper bzw. Antikörperfragment um einen rekombinanten Antikörper bzw. ein rekombinantes Antikörperfragment handelt.

5. Antikörper oder Antikörperfragment nach einem der vorhergehenden Ansprüche zur Verwendung bei der Behandlung einer entzündlichen Erkrankung oder von Krebs.

6. Nukleinsäurezusammensetzung, die eine Nukleinsäuresequenz oder mehrere Nukleinsäuresequenzen umfasst, die den Antikörper oder das Antikörperfragment nach einem der vorhergehenden Ansprüche codiert bzw. codieren.

7. Vektorzusammensetzung, die einen Vektor oder mehrere Vektoren umfasst, der bzw. die die Nukleinsäuresequenz oder mehreren Nukleinsäuresequenzen gemäß Anspruch 7 umfasst bzw umfassen.

8. Zelle, die die Vektorzusammensetzung gemäß Anspruch 8 umfasst.

9. Pharmazeutische Zusammensetzung, die einen Antikörper bzw. ein Antikörperfragment nach einem der Ansprüche 1 bis 6 und einen pharmazeutisch unbedenklichen Träger oder Exzipienten umfasst.

## Revendications

1. Anticorps ou fragment d'anticorps spécifique pour IL-17C humaine, ledit anticorps ou fragment d'anticorps se liant de manière bivalente à un homodimère d'IL-17C et formant un complexe constitué dudit anticorps ou fragment d'anticorps et d'un homodimère d'IL-17C, et l'anticorps ou le fragment d'anticorps se liant à la région PVLRPEEVL (SEQ ID NO: 27) sur IL-17C et ledit anticorps ou fragment d'anticorps bloquant la liaison d'IL-17C à IL17RE.

2. Anticorps ou fragment d'anticorps selon l'une quelconque des revendications précédentes, ledit anticorps ou fragment d'anticorps étant un anticorps ou fragment d'anticorps monoclonal.

3. Anticorps ou fragment d'anticorps selon l'une quelconque des revendications précédentes, ledit anticorps ou fragment d'anticorps étant un anticorps ou fragment d'anticorps isolé.

4. Anticorps ou fragment d'anticorps selon l'une quelconque des revendications précédentes, ledit anticorps ou fragment d'anticorps étant un anticorps ou fragment d'anticorps recombinant.

5. Anticorps ou fragment d'anticorps selon l'une quelconque des revendications précédentes pour une utilisation dans le traitement d'un trouble inflammatoire ou d'un cancer.

6. Composition d'acide nucléique comprenant une séquence d'acides nucléiques ou une pluralité de séquences d'acides nucléiques codant pour l'anticorps ou le fragment d'anticorps selon l'une quelconque des revendications précédentes.

7. Composition de vecteur comprenant un vecteur ou une pluralité de vecteurs comprenant la séquence d'acides nucléiques ou la pluralité de séquences d'acides nucléiques selon la revendication 7.

8. Cellule comprenant la composition de vecteur selon la revendication 8.

9. Composition pharmaceutique comprenant un anticorps ou fragment d'anticorps selon l'une quelconque des revendications 1 à 6 et un support ou excipient pharmaceutiquement acceptable.
